# EUROPEAN PATENT SPECIFICATION

(11) **EP 2 967 496 B1**
(45) Date of publication and mention of the grant of the patent: **29.04.2020**
(21) Application number: 14764920.6
(22) Date of filing: 14.03.2014
(51) Int. Cl.: A61B 8/12, A61B 8/00, A61B 8/14, G10K 11/35, A61B 8/08, A61B 90/00

(54) **ACTIVE LOCALIZATION AND VISUALIZATION OF MINIMALLY INVASIVE DEVICES USING ULTRASOUND**
AKTIVE LOKALISIERUNG UND VISUALISIERUNG VON MINIMAL INVASIVEN VORRICHTUNGEN MITTELS ULTRASCHALL
LOCALISATION ET VISUALISATION ACTIVES DE DISPOSITIFS MINI-INVASIFS À L'AIDE D'ULTRASONS

(30) Priority: 15.03.2013 US 201361801502 P
(43) Date of publication of application: 20.01.2016
(73) Proprietor: CONAVI MEDICAL INC., Toronto ON M3B 2V1 (CA); Sunnybrook Research Institute, Toronto, Ontario M4N 3M5 (CA)
(72) Inventor: COURTNEY, Brian, Toronto, Ontario M4S 1E2 (CA); LUKACS, Marc, Toronto, Ontario M5V 3N3 (CA); THIND, Amandeep, Toronto, Ontario M2L 2S8 (CA)
(74) Representative: Dennemeyer & Associates S.A.
(86) International application number: PCT/CA2014/050251
(87) International publication number: WO 2014/139005

(56) References cited:
- WO-A1-2011/138698
- WO-A1-2012/172458
- WO-A1-2013/005123
- US-A- 4 407 294
- US-A- 6 045 508
- US-A1- 2003 013 958
- US-A1- 2004 015 075
- US-A1- 2011 172 530
- US-A1- 2012 197 113
- US-B2- 6 587 709

## Description

### BACKGROUND

The present disclosure relates generally to the field of image guidance of minimally invasive procedures within mammals.

Minimally invasive diagnostic and therapeutic procedures are commonplace in modern medicine. Cardiovascular medicine provides several examples where minimally invasive procedures are an integral part of patient care. Example procedures include electrophysiology studies to better diagnose or characterize arrhythmias, ablation procedures to treat arrhythmias, angioplasty and stenting for coronary artery disease, minimally invasive valve replacement or repair and several others. Many of these procedures involve the introduction of devices into the chambers, potential spaces (such as the pericardial space), vessels and tissues of mammalian anatomy.

Several imaging modalities or other guidance systems are used to guide the manipulation and placement of devices during minimally invasive procedures, including x-rays, ultrasound, MRI, optical imaging techniques and electroanatomical mapping systems (such as CARTO™).

Ultrasound can be a particularly attractive imaging modality for many minimally invasive procedures as it provides real-time images of anatomy with better soft-tissue contrast and cross-sectional imaging than is readily available with x-ray based techniques such as fluoroscopy. Furthermore, ultrasound does not use ionizing radiation, which has been shown to have risks associated with it, such as an increased risk of malignancy.

However, ultrasound is subject to some artifacts that can impede its use in image guidance. Such artifacts include the variability in the visibility of devices in the field of view based on their alignment with ultrasound beams used for imaging, the reflectivity of the materials of the devices being imaged, the surface texture of the devices being imaged and other factors well known in the art of ultrasound imaging. For example, a polished needle in the field of view of an ultrasound probe will typically be displayed as a bright line if the ultrasound beam is perpendicular to a portion of the surface of the needle, as the needle is very reflective and the perpendicular orientation would cause a relatively large portion of the ultrasound imaging energy to reflect back towards the imaging probe. That same needle would typically not be as visible if the needle were oriented such that the reflected ultrasound imaging beam was directed away from the imaging probe. Devices that have a more coarsely textured surface or include other features that cause incident ultrasound energy to be scattered rather than reflected in a specular fashion, will typically have less angle-dependency in terms of the intensity of their appearance in an ultrasound image. In some cases, devices can appear to be in locations where they don't truly reside due to artifacts caused by multiple reflections of ultrasound beams off of surrounding structures.

Ultrasound provides 'real-time' imaging system with a limited field of view over a short period of time. It takes more time to scan a region (such as a 2D plane or contoured 2D surface, or a 3D volume) in order to generate an image. In comparison, fluoroscopy can collect an image over a wide field of view, wherein the pixels in the image are collected in parallel, providing a high degree of temporal resolution and a large field of view. One of the challenges of using ultrasound for image guidance relates to keeping the devices or anatomy being imaged within the field of view that is being interrogated by the ultrasound device itself. Generally speaking, enlarging the field of view imaged with ultrasound often involves reducing the frequency at which the imaging dataset can be refreshed, as there are lower limits as to how much time is required to wait for ultrasound energy beams to propagate repeatedly through tissue with adequate sampling of the region being imaged.

US 6,587,709 (Solf et al.) discloses an imaging ultrasound system with an ultrasound transducer and an image processing unit for the acquisition of a three-dimensional ultrasound image of the body of a patient, and a catheter for carrying out a therapeutic or diagnostic intervention in the body of the patient. The catheter accommodates, in addition to the customary instruments necessary to carry out its task, three ultrasound receivers that are mounted at a distance from one another on the tip of the catheter and are capable of detecting the arrival of scan signals of the ultrasound transducer. The distance between the ultrasound transducer and the receivers can be calculated from the transit time of the scan signals. The receivers can thus be localized in space. Localization enables notably the selection of, for example, the plane from the three-dimensional ultrasound data that contains all three receivers of the catheter. The tip of the catheter can thus be automatically tracked and displayed on a monitor at all times without manual displacement of the ultrasound transducer being necessary.

### SUMMARY

Systems and methods are provided for localizing and visualizing devices with the use of an intracorporeal ultrasound imaging probe during a medical procedure. A primary intracorporeal ultrasound imaging probe is employed to image a three-dimensional region via scanning, and to locate a secondary intracorporeal device having one or more ultrasonic beacon transducers.

When an A-scan vector associated with the primary intracorporeal ultrasound imaging device is directed towards one or more of the ultrasound beacon transducers of the secondary intracorporeal device, a communication signal is transmitted from the secondary intracorporeal device to a control and processing system associated with the primary intracorporeal ultrasound imaging device, either through acoustic transmission or non-acoustic transmission. Example embodiments are provided in which acoustic communication may be employed within the imaging band, or in a separate communication band distinct from the communication band. Various example dual-band, single-stack ultrasound imaging transducer embodiments are disclosed.

Accordingly, in one aspect, there is provided a method of locating a secondary intracorporeal device while performing imaging with an intravascular or intracardiac imaging catheter, wherein the imaging catheter comprises an ultrasonic imaging device, and wherein the imaging catheter is configured for three-dimensional scanning, and wherein the secondary intracorporeal device comprises one or more ultrasonic beacon transducers having a combined broad angular response, the method comprising, after performing a scanning operation with the and detect signals associated with received ultrasound imaging energy and communication signals emitted by one or more of the ultrasound beacon transducers:
processing signals associated with the received ultrasonic imaging energy to generate an image; and
   processing the signals to identify a communication signal that was received, when a given imaging A-scan vector was directed towards a given ultrasound beacon transducer on the secondary intracorporeal device; and
processing the communication signal to locate the secondary intracorporeal device relative to the imaging catheter, based on the direction of the given imaging A-scan vector and a time delay associated with the communication signal.

In another aspect, there is provided a method of locating a secondary intracorporeal device while performing imaging with a primary intracorporeal ultrasonic imaging probe, wherein the primary intracorporeal ultrasonic imaging probe comprises an ultrasonic imaging device having an imaging frequency band and a communication frequency band, wherein the primary intracorporeal ultrasonic imaging probe is configured for three-dimensional scanning, and wherein the secondary intracorporeal device comprises one or more ultrasonic beacon transducers having a combined broad angular response, the method comprising:
controlling the primary intracorporeal ultrasonic imaging probe to scan a three-dimensional imaging volume, such that:
   the ultrasonic imaging device emits ultrasonic imaging energy and receives received ultrasonic imaging energy at a plurality of imaging A-scan vectors spanning the three-dimensional imaging volume, wherein the ultrasonic imaging energy lies within the imaging frequency band; and
   the ultrasonic imaging device emits ultrasonic communication energy at a plurality of communication A-scan vectors spanning a three-dimensional communication volume, wherein the ultrasonic communication energy lies within the communication frequency band;
receiving, when a given communication A-scan vector is directed towards a given ultrasonic beacon transducer on the secondary intracorporeal device, a communication signal associated with the given ultrasound beacon transducer;
processing imaging signals associated with the received ultrasonic imaging energy to generate an image; and
processing the communication signal to locate the secondary intracorporeal device relative to the primary intracorporeal ultrasonic imaging probe, based on the direction of the given communication A-scan vector and a time delay associated with the communication signal.

In another aspect, there is provided an intracorporeal ultrasonic imaging system comprising:
an intravascular or intracardiac imaging catheter comprising an ultrasonic imaging device, wherein said imaging catheter is configured for scanning a three-dimensional imaging volume;
a secondary intracorporeal device comprising one or more ultrasonic beacon transducers having a combined broad angular response;
a control and processing system interfaced with said imaging catheter, said control and processing system comprising one or more processors and memory coupled to said one or more processors, said memory storing instructions, which,
when executed by said one or more processors, causes said one or more processors to perform operations comprising:
controlling said imaging catheter to scan the three-dimensional imaging volume such that said ultrasonic imaging device emits ultrasonic imaging energy and receives ultrasonic imaging energy at a plurality of imaging A-scan vectors spanning the three-dimensional imaging volume;
receiving, when a given imaging A-scan vector is directed towards a given ultrasonic beacon transducer on said secondary intracorporeal device, a communication signal associated with said given ultrasonic beacon transducer;
processing imaging signals associated with the received ultrasonic imaging energy to generate an image; and
processing the communication signal to locate said secondary intracorporeal device relative to said imaging catheter, based on the direction of the given imaging A-scan vector and a time delay associated with the communication signal.

In another aspect, there is provided an intracorporeal ultrasonic imaging system comprising:
a primary intracorporeal ultrasonic imaging probe comprising an ultrasonic imaging device, wherein said primary intracorporeal ultrasonic imaging probe is configured for scanning a three-dimensional imaging volume;
a secondary intracorporeal device comprising one or more ultrasonic beacon transducers having a combined broad angular response;
a control and processing system interfaced with said primary intracorporeal ultrasonic imaging probe, said control and processing system comprising one or more processors and memory coupled to said one or more processors, said memory storing instructions, which, when executed by said one or more processors, causes said one or more processors to perform operations comprising:
   controlling said primary intracorporeal ultrasonic imaging probe to scan the three-dimensional imaging volume, such that:
   said ultrasonic imaging device emits ultrasonic imaging energy and receives received ultrasonic imaging energy at a plurality of imaging A-scan vectors spanning the three-dimensional imaging volume, wherein the ultrasonic imaging energy lies within the imaging frequency band; and
   said ultrasonic imaging device emits ultrasonic communication energy at a plurality of communication A-scan vectors spanning a three-dimensional communication volume, wherein the ultrasonic communication energy lies within the communication frequency band;
   receiving, when a given communication A-scan vector is directed towards a given ultrasonic beacon transducer on said secondary intracorporeal device, a communication signal associated with said given ultrasonic beacon transducer;
   processing imaging signals associated with the received ultrasonic imaging energy to generate an image; and
   processing the communication signal to locate said secondary intracorporeal device relative to said primary intracorporeal ultrasonic imaging probe, based on the direction of the given communication A-scan vector and a time delay associated with the communication signal.

In another aspect, there is provided an ultrasonic transducer having a frequency response comprising a first frequency band and a second frequency band, comprising:
a backing layer;
an inactive piezoelectric layer contacting said backing layer;
a lower electrode layer contacting said inactive piezoelectric layer;
an active piezoelectric layer contacting said lower electrode;
an upper electrode layer contacting said active piezoelectric layer; and
one or more matching layers contacting said upper electrode layer;
wherein the thicknesses of said active piezoelectric layer and said inactive piezoelectric layer are selected to control the frequency separation of the first frequency band and the second frequency band.

In another aspect, there is provided an ultrasonic transducer comprising:
a backing layer;
a first lower electrode layer contacting said backing layer;
a first active piezoelectric layer contacting said first lower electrode layer;
a first upper electrode contacting said first active piezoelectric layer;
a first matching layer contacting said first upper electrode;
a second lower electrode contacting said first matching layer;
a second active piezoelectric layer contacting said second lower electrode;
a second upper electrode contacting said second active piezoelectric layer; and
a second matching layer contacting said second upper electrode;
wherein said first active piezoelectric layer has a thickness suitable for defining a first frequency band;
wherein said second active piezoelectric layer has a thickness suitable for defining a second frequency band;
wherein said first matching layer has a thickness approximately equal to a quarter of an operative wavelength associated with the first frequency band; and
wherein said second active piezoelectric layer and said second matching layer together have a thickness approximately equal to a quarter of an operational wavelength associated with the first frequency band, such that said first matching layer, said second active piezoelectric layer and said second matching layer act as matching layers for said first active piezoelectric layer.

In another aspect, there is provided a method of locating a secondary intracorporeal device while performing imaging with a primary intracorporeal imaging probe, wherein the primary intracorporeal imaging probe comprises an ultrasound device, and wherein the primary intracorporeal imaging probe is configured for three-dimensional scanning, and wherein the secondary intracorporeal device comprises one or more ultrasonic beacon transducers having a combined broad angular response, the method comprising:
controlling the primary intracorporeal imaging probe to scan a three-dimensional imaging volume such that imaging energy is emitted and received at a plurality of imaging A-scan vectors spanning the three-dimensional imaging volume;
controlling the ultrasound device to emit ultrasound energy at a plurality of the imaging A-scan vectors;
receiving, when a given imaging A-scan vector is directed towards a given ultrasonic beacon transducer on the secondary intracorporeal device, a communication signal associated with the given ultrasound beacon transducer;
generating an image based on the received imaging energy; and
processing the communication signal to locate the secondary intracorporeal device relative to the primary intracorporeal imaging probe, based on the direction of the given imaging A-scan vector and a time delay associated with the communication signal.

A further understanding of the functional and advantageous aspects of the disclosure can be realized by reference to the following detailed description and drawings.

### BRIEF DESCRIPTION OF THE DRAWINGS

Embodiments will now be described, by way of example only, with reference to the drawings, in which:
**FIG. 1** illustrates the localization of a secondary intracorporeal device via a primary intracorporeal ultrasonic imaging probe.
**FIG. 2** is a flow chart illustrating an example method of localizing a secondary intracorporeal device via a primary intracorporeal ultrasonic imaging probe.
**FIG. 3** is a block diagram showing an example system for localizing and visualizing one or more secondary intracorporeal device via a primary intracorporeal ultrasonic imaging probe.
**FIG. 4** is a perspective drawing of a flexible imaging probe with an adapter, conduit, and imaging assembly.
**FIG. 4A** is a cross sectional view of the mid-section of the imaging probe of FIG. 4 taken along the dotted line.
**FIG. 4B** is a magnified and expanded drawing of the distal region of the imaging probe of FIG. 4.
**FIGS. 5A - 5K** describe embodiments of techniques for causing tilting of a tiltable member.

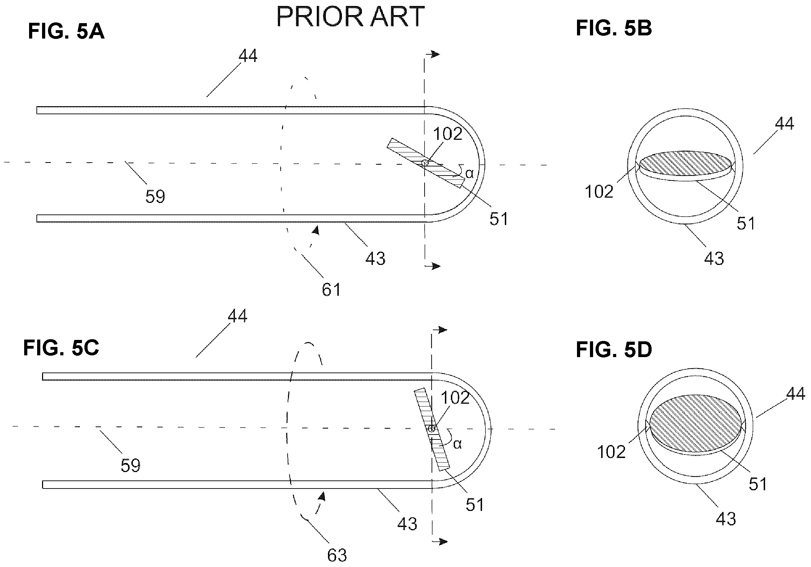

**FIG. 5A** shows a longitudinal cutaway of a catheter in which the tilting is caused by centripetal motion.
**FIG. 5B** shows a cross-sectional cutaway of the catheter shown in FIG. 5A.
**FIG. 5C** shows the catheter of FIG. 5A and the resulting tilting caused by rotating the scanning assembly at a faster rate than that of FIG. 5A.
**FIG. 5D** shows a cross-sectional cutaway of the catheter shown in FIG. 5B.

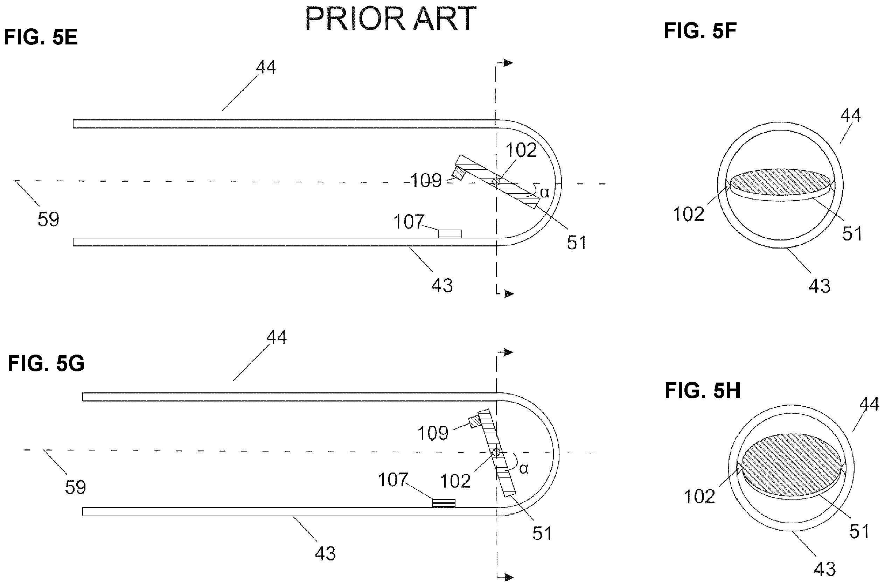

**FIG. 5E** shows a longitudinal cutaway of a catheter in which the tilting is controlled using one or more magnets.
**FIG. 5F** shows a cross-sectional cutaway of the catheter in FIG. 5E.
**FIG. 5G** shows the catheter of FIG. 5E and the resulting deflection caused by magnetism.
**FIG. 5H** shows a cross-sectional cutaway of the catheter in FIG. 5G.

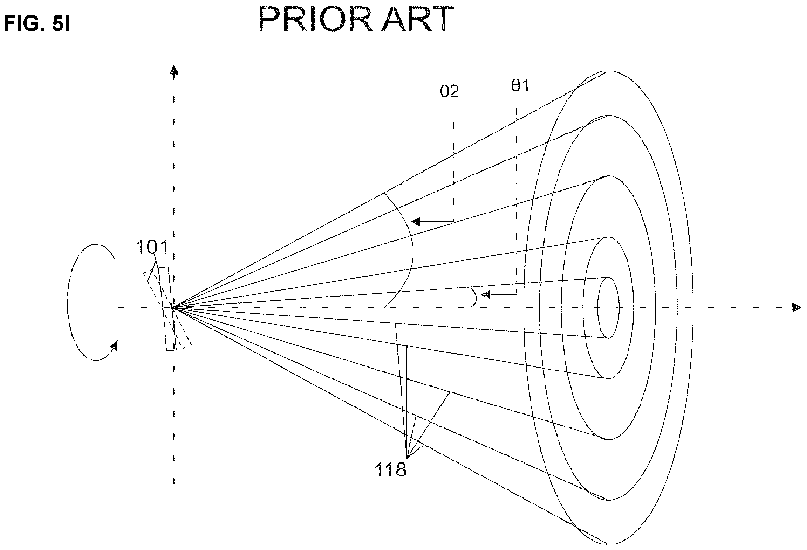

**FIG. 5I** shows a potential scanning pattern for generating 3D images with imaging angle information.

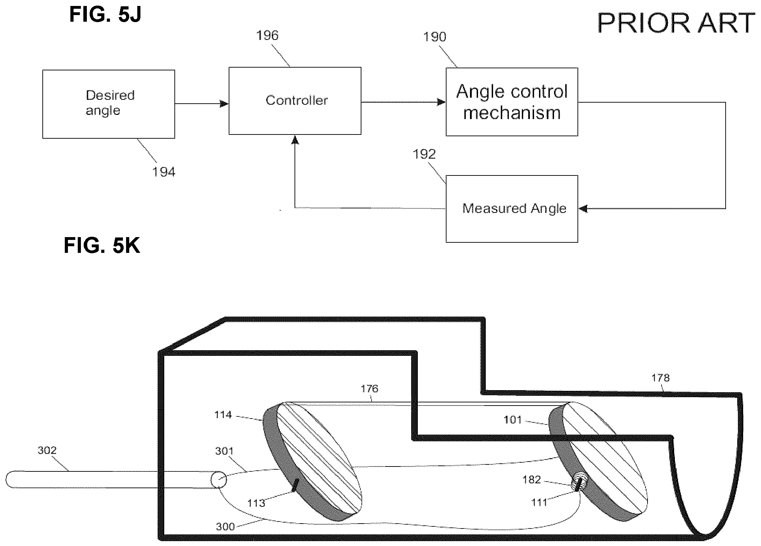

**FIG. 5J** illustrates a control system in which the angle sensing transducer is employed to provide feedback for controlling a direction of the emitted imaging beam.
**FIG. 5K** shows an implementation of a system using a torsional spring as a restoring mechanism.

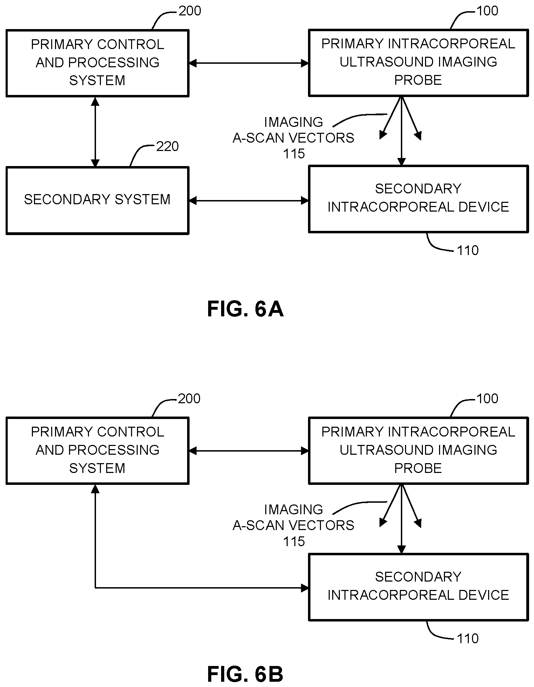

**FIGS. 6A-6D** are block diagrams illustrating various system configurations, involving acoustic and non-acoustic return communication signals.

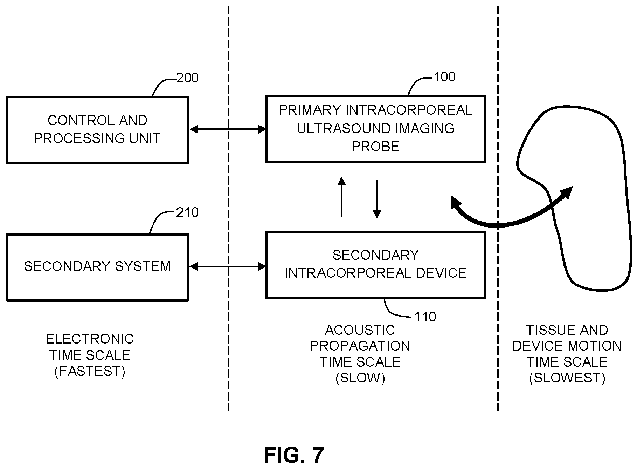

**FIG. 7** illustrates the temporal relationship between the electronic interactions, the acoustic interactions and the environmental and use interactions of the primary and secondary device systems.

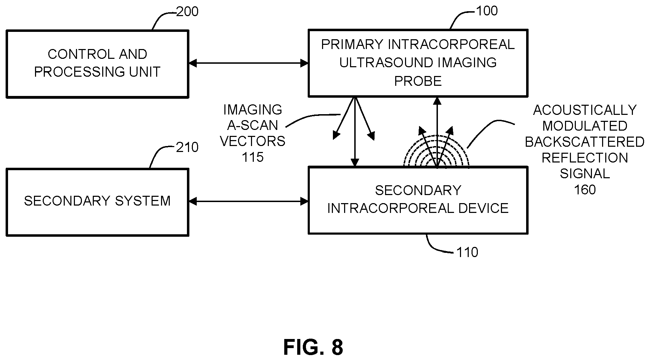

**FIG. 8** is block diagram illustrating communication via an echo-backscatter mode.

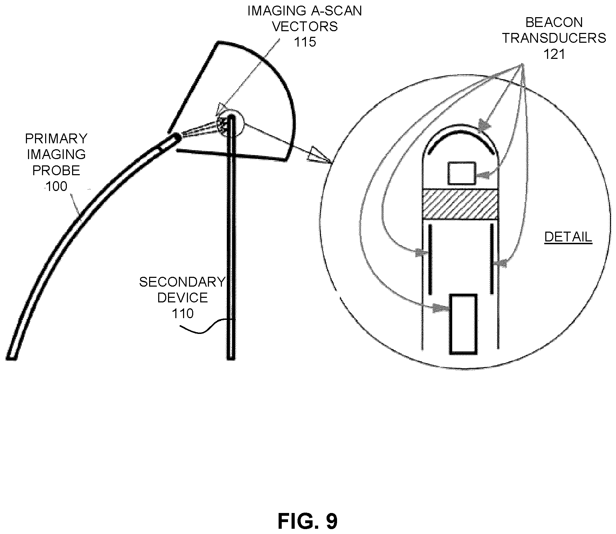

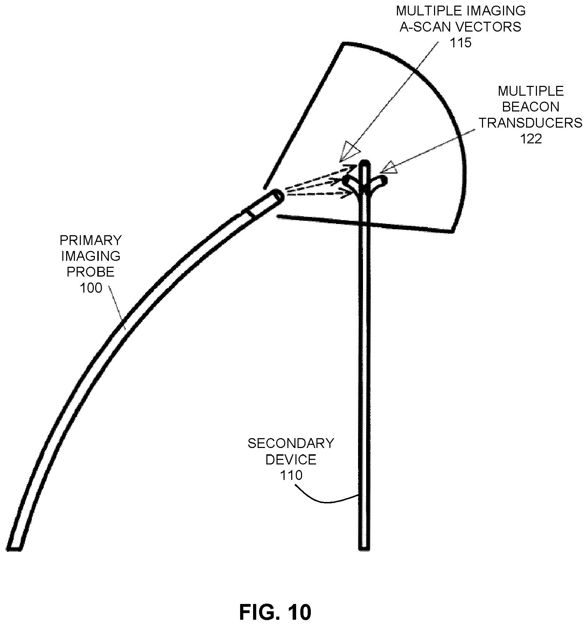

**FIGS. 9** and **10** illustrate the localization of a secondary intracorporeal device via a primary intracorporeal ultrasonic imaging probe involving a plurality of beacon transducers.

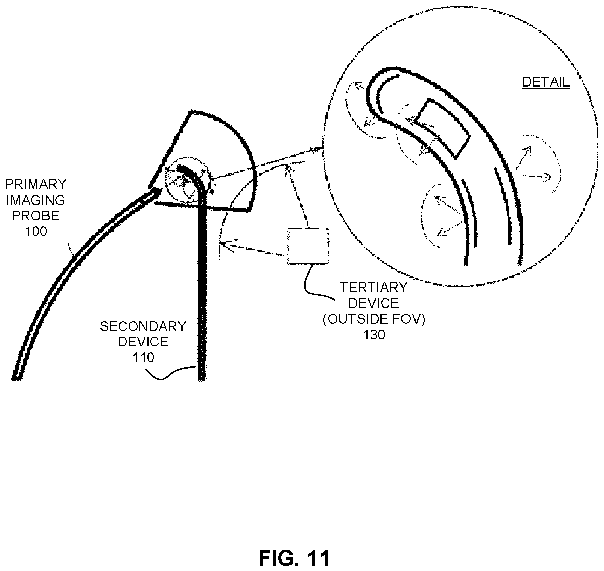

**FIG. 11** illustrates an example embodiment involving multiple beacon transducers for determining the orientation of a secondary device where the return acoustic signal is represented as a diffracting wave as opposed to only representing the direction of the A-scan vector.

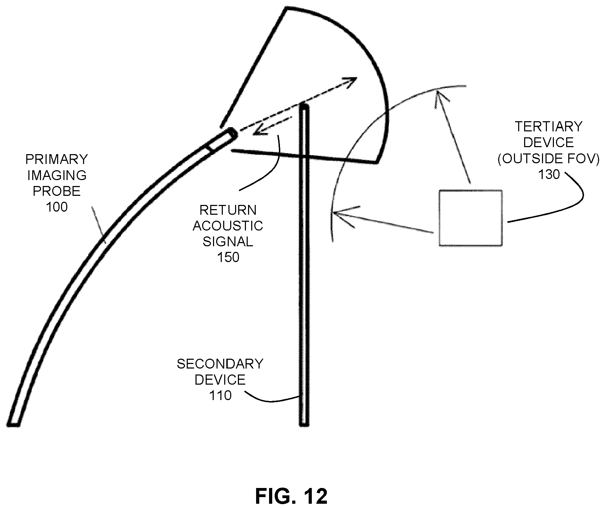

**FIG. 12** illustrates an example embodiment in which a tertiary device is employed for locating the primary imaging probe and secondary device relative to an external frame of reference.

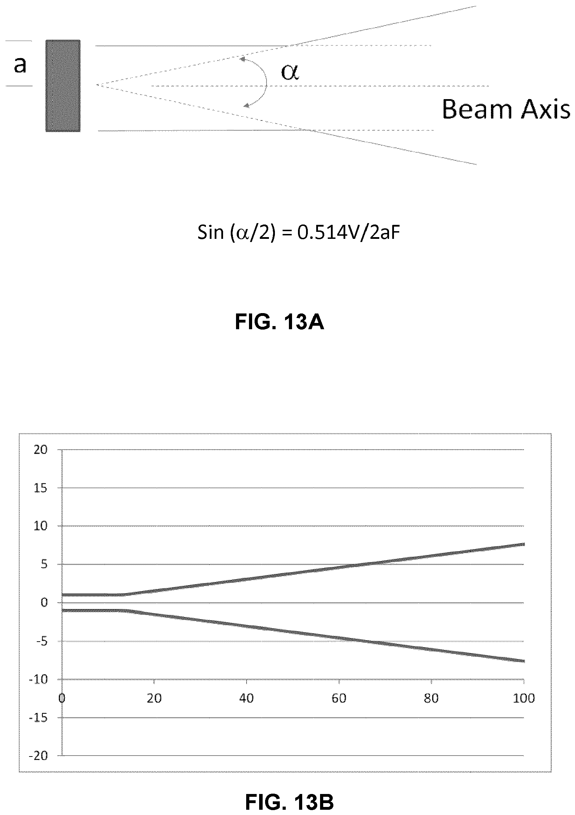

**FIG. 13A** is an illustration of the approximate -6dB beam profile for a single element of a given aperture size. For example purposes, the aperture is considered circular.
**FIG. 13B** is a plot of the -6 dB beam profile of a 10 MHz transducer with a 2mm diameter aperture. All axes are in mm.

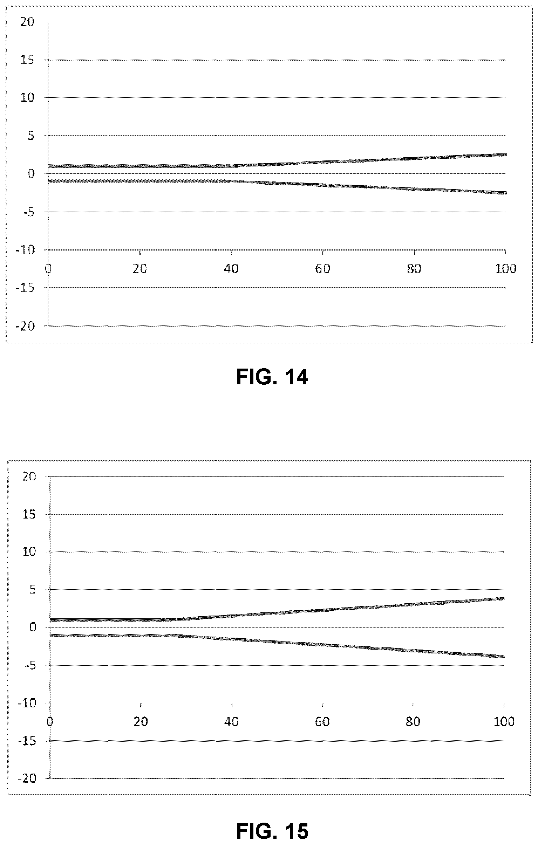

**FIG. 14** is a plot of the -6 dB beam profile of a 30 MHz transducer with a 2mm diameter aperture. All axes are in mm.
**FIG. 15** is a plot of the -6 dB beam profile of a 20 MHz transducer with a 2mm diameter aperture. All axes are in mm. Beam profile at 6cm in depth is 4.6mm in diameter.

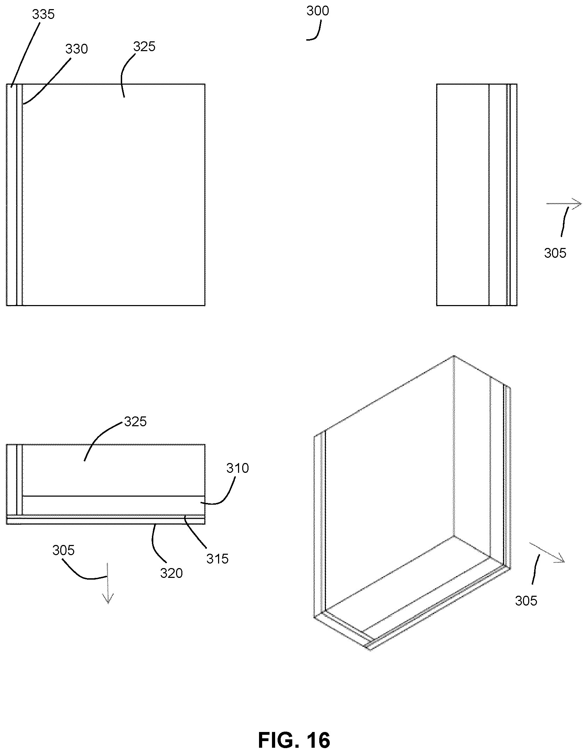

**FIG. 16** provides an isometric schematic of a square shaped aperture for a baseline imaging transducer stack.

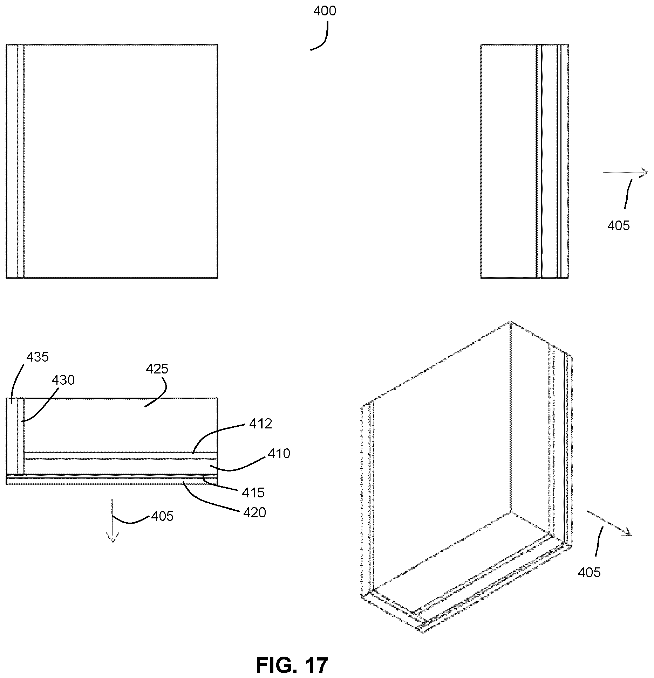

**FIG. 17** provides an isometric schematic of a square shaped aperture for a modified imaging transducer stack.

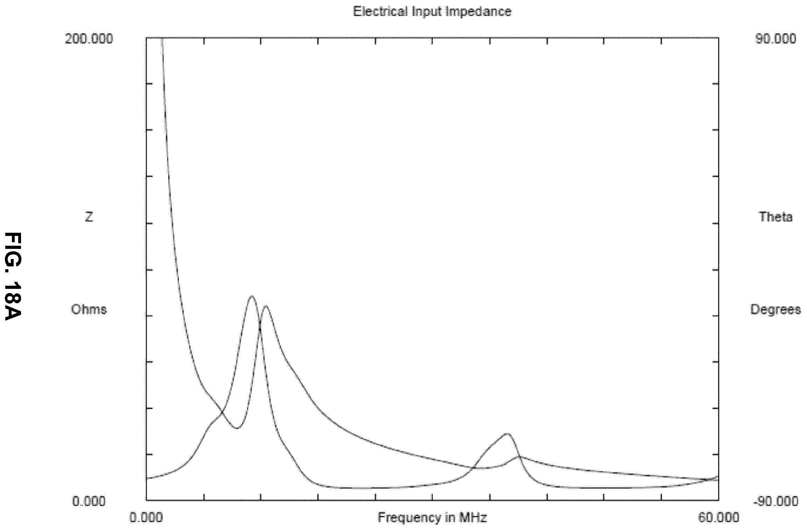

**FIG.18A** plots the simulated electrical impedance of the baseline imaging transducer stack and the modified imaging transducer stack.

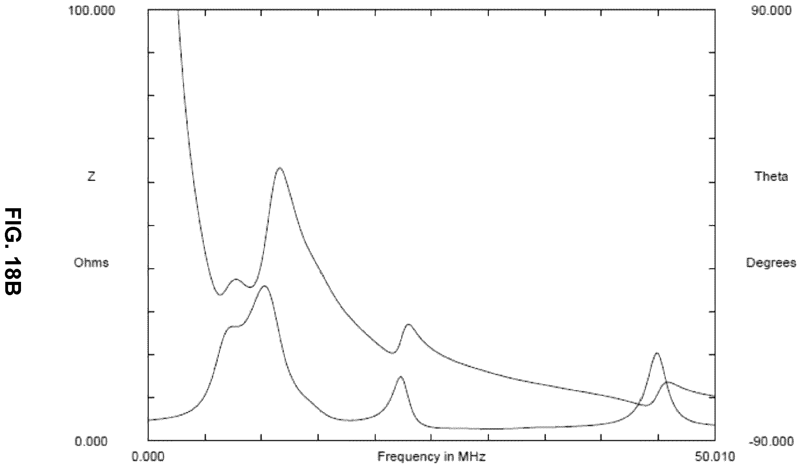

**FIG.18B** plots the simulated two-way excitation response of a baseline imaging transducer stack and a modified imaging transducer stack.

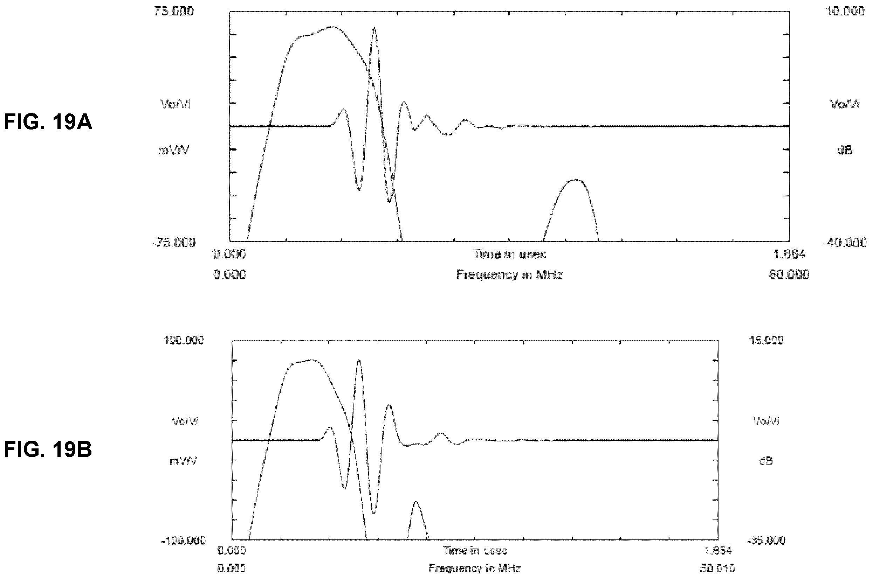

**FIG. 19A-19B** provides a comparison of the one-way transmit excitation response of the baseline imaging transducer stack and the modified imaging transducer stack respectively.

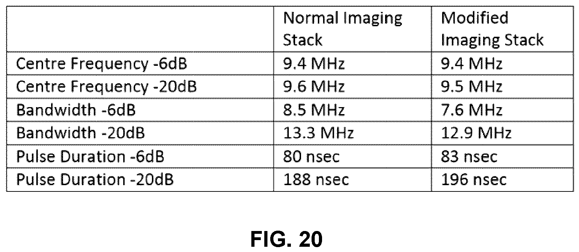

**FIG. 20** provides a comparison of one-way acoustic parameters that define the pulse excitation response of the baseline imaging transducer stack and the modified imaging transducer stack shown in FIG. 19A-19B.

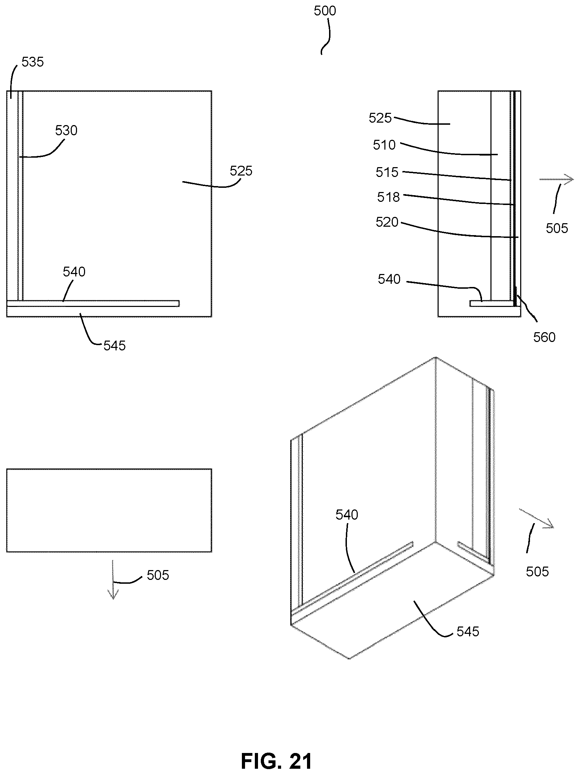

**FIG. 21** shows an isometric schematic of a square shaped aperture for the exemplary modified dual piezoelectrically active transducer stack.

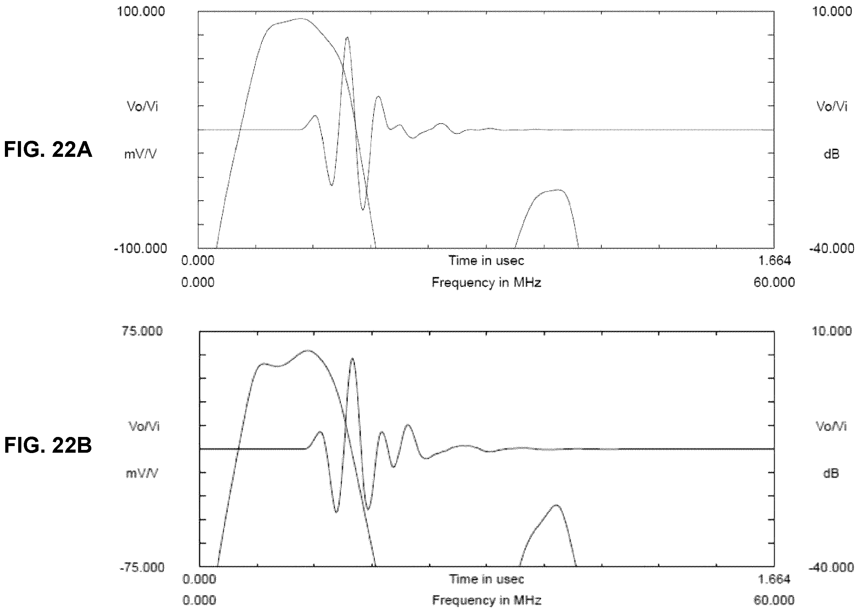

**FIGS. 22A** and **22B** plot the simulated two-way excitation response of the baseline imaging transducer stack and the dual piezoelectrically active transducer stack.

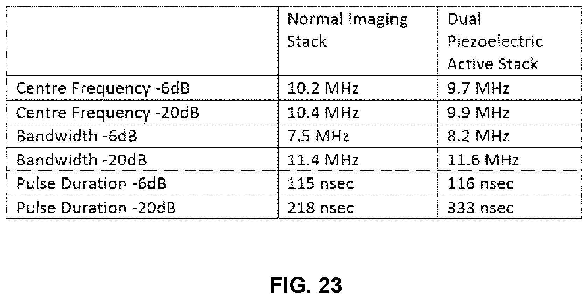

**FIG. 23** provides a comparison of the simulated two-way transmit excitation response of a second baseline imaging transducer stack and a dual piezoelectrically active transducer stack.

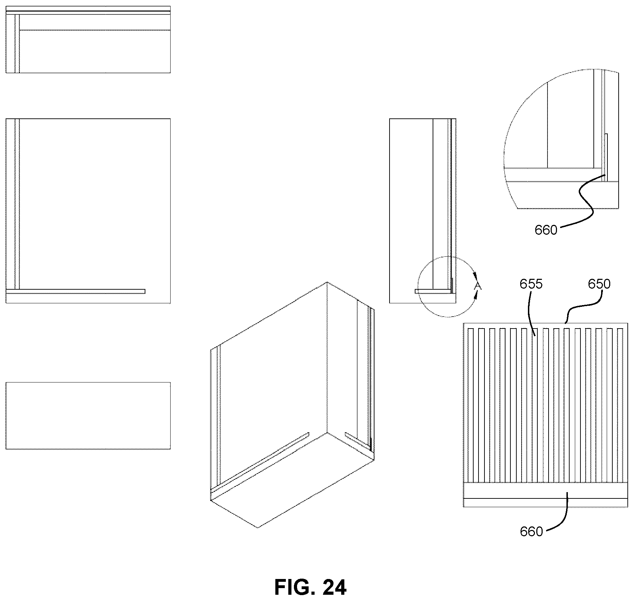

**FIG. 24** shows an isometric schematic of a square shaped aperture for the exemplary modified dual piezoelectrically active transducer stack similar to that shown in FIG. 21 with an additional TOP view of the transducer stack shown without the top matching layer to expose an exemplary electrode pattern that defines a single element diffraction grating transducer.

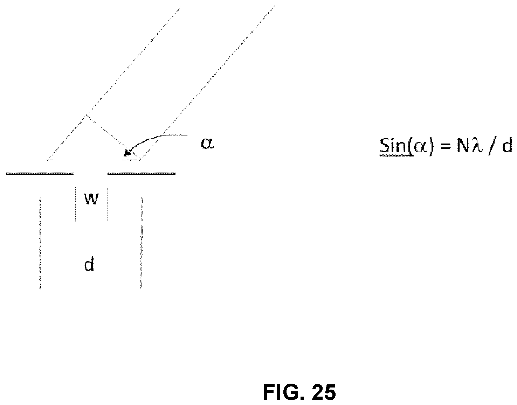

**FIG. 25** illustrates the conditions for constructive interference for a diffraction grating.

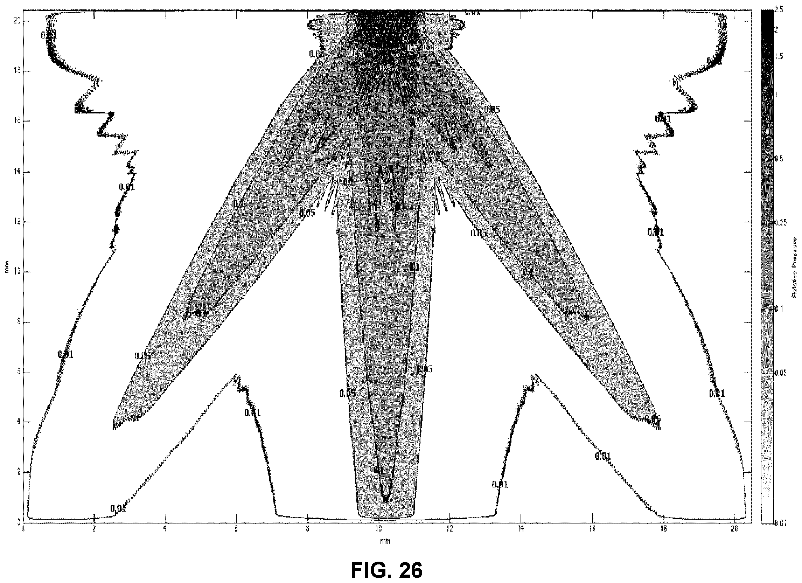

**FIG. 26** shows the results of a linear 2D propagation simulation plotting the maximum temporal intensity along the perpendicular plane that bisects the elements of the grating structure.

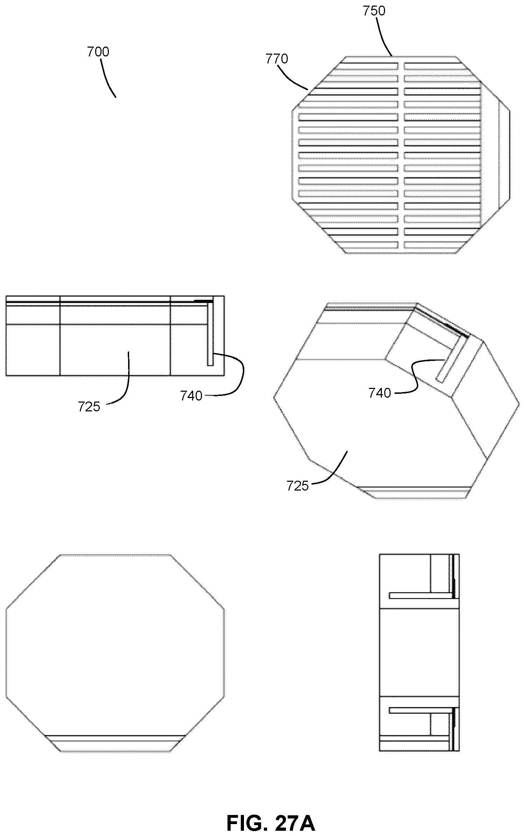

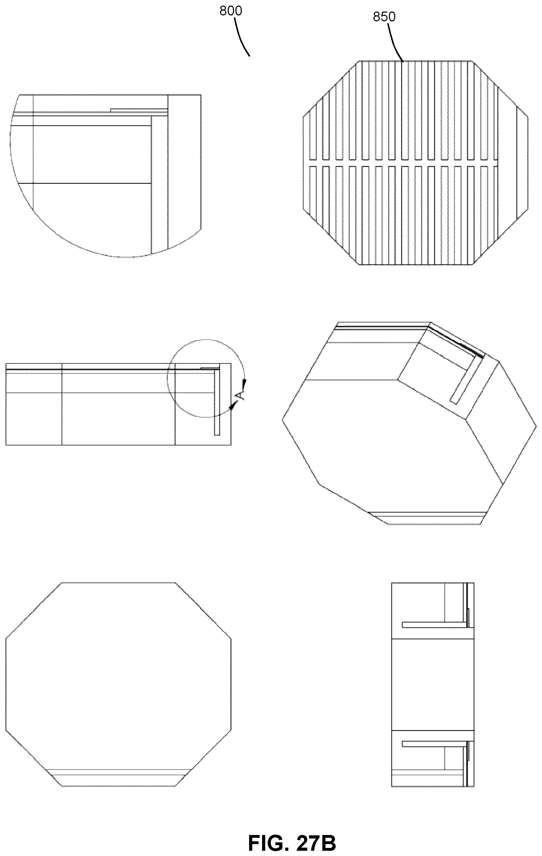

**FIGS. 27A** and **27B** show isometric schematics of different configurations of a shaped aperture for the exemplary modified dual piezoelectrically active transducer stack that has been shaped into an 8 sided structure.

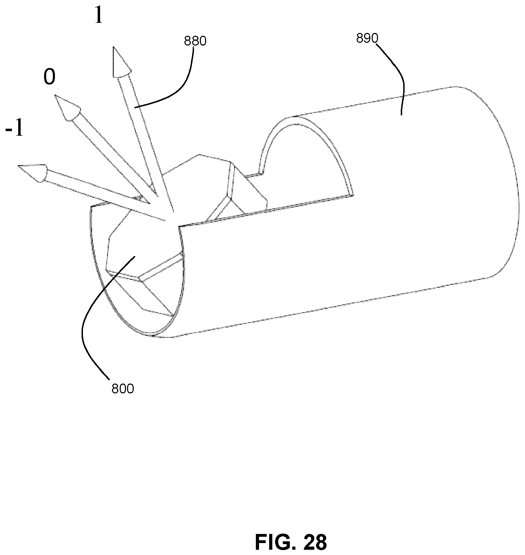

**FIG. 28** shows a perspective view of the -1,0,1 grating lobes of a diffraction grating integrated into an imaging transducer where the elevation axis of the diffraction grating transducer is perpendicular to the tilt axis of the transducer. The tilt axis is a relative reference frame with respect to the longitudinal axis of the catheter.

### DETAILED DESCRIPTION

Various embodiments and aspects of the disclosure will be described with reference to details discussed below. The following description and drawings are illustrative of the disclosure and are not to be construed as limiting the disclosure. Numerous specific details are described to provide a thorough understanding of various embodiments of the present disclosure. However, in certain instances, well-known or conventional details are not described in order to provide a concise discussion of embodiments of the present disclosure.

As used herein, the terms, "comprises" and "comprising" are to be construed as being inclusive and open ended, and not exclusive. Specifically, when used in the specification and claims, the terms, "comprises" and "comprising" and variations thereof mean the specified features, steps or components are included. These terms are not to be interpreted to exclude the presence of other features, steps or components.

As used herein, the term "exemplary" means "serving as an example, instance, or illustration," and should not be construed as preferred or advantageous over other configurations disclosed herein.

As used herein, the terms "about" and "approximately" are meant to cover variations that may exist in the upper and lower limits of the ranges of values, such as variations in properties, parameters, and dimensions. Unless otherwise specified, the terms "about" and "approximately" mean plus or minus 20 percent or less.

Unless defined otherwise, all technical and scientific terms used herein are intended to have the same meaning as commonly understood to one of ordinary skill in the art.

Various embodiments described herein refer to an "operational wavelength" of a transducer. The term "operational wavelength" may be defined as described below.

When designing a piezoelectric based ultrasound transducer stack, the thickness of the active layer is often substantially smaller than the width of the active layer (typically 1/10th the size or smaller). This is done to separate the frequency of the fundamental thickness resonant mode of the layer from any lateral resonance mode and/or to maintain beam characteristics for imaging. Within any propagating material or medium (such as conductive silver epoxy or human tissue) the propagating waveform will have a fundamental wavelength that is related to the frequency of the fundamental resonance mode through the speed of sound of the material or medium, according to the relation: Wavelength = speed of sound / frequency. In some embodiments, the operational wavelength may be this fundamental design wavelength.

In real transducers, materials are not perfect (ideal) resonators and therefore the fundamental frequency is actually a band of excited frequencies that can be characterized by a center frequency and a bandwidth of excited frequencies. Matching layers and backing layers are added to effectively couple as much of the resonant energy out the front face of the transducer stack and into the propagating medium in as short a time as possible. This will result in yet a broader frequency response of the stack, (i.e. broader bandwidth of excited frequencies) allowing for the transducer stack to more closely replicate an ultrasound pulse response waveform from a short excitation transmit signal (say a single cycle waveform) as well as from a more narrow band excitation pulse such as a tone burst of several cycles in duration. Fabrication tolerances can also result in deviations of the time and frequency response of the transducer. In some embodiments, the operational wavelength associated with the transducer may be the wavelength within the frequency response of the stack, such as the center wavelength. For example, in some embodiments, the operational wavelength associated with the transducer may include any wavelength within this combined design, excitation pulse, fabrication tolerance dependent bandwidth. It will be understood that the term wavelength is dependent on the material through which the ultrasound energy is propagating, and thus refers to the wavelength within a particular material.

In many clinical applications, it is important to be able to accurately track the position of all or a portion of one or more devices within an anatomic region being imaged. For example, the position of the tip of an ablation catheter relative to the anatomy of the left atrium plays an important role in whether or not an ablation procedure is successful. If the tip is not making adequate contact with the tissue targeted for ablation, the result may be an unsuccessful treatment. Similarly, if the tip of an ablation catheter is inserted deeply within a pulmonary vein, rather than near the ostium of a pulmonary vein, there is an increased risk of causing pulmonary vein stenosis during ablation.

As noted above, various imaging modalities may be employed to track the position of a device within a subject. One option to locate an intracorporeal device, such as a catheter, is to use ultrasound signals. For example, one known method of locating an intracorporeal device via ultrasound location sensing involves the use an external ultrasound device that is contacted with the body in order to locate the intracorporeal device having a series of spatially separated ultrasonic beacon transducers, as described in US Patent No. 6,587,709 (Solf) and in US Patent No. 4,249,539 (Vilkomerson et al.).

According to another approach, as described in US Patent Publication No. 2013/0217997 (Byrd), an ultrasound catheter may be located using time-of-flight information associated with non-directional, or weakly directional, ultrasound signals sent between the ultrasound catheter, and ultrasound beacons residing on neighbouring positioning catheters, where the ultrasound catheter employs at least one non-directional ultrasound transducer for communicating with beacon transducers on the positioning probes.

Unfortunately, such methods are prone to problems associated with the need for external imaging devices, multiple probes, complex beacon geometries and configurations, limited location resolution and accuracy, and artifacts associated with scattering and variations in the speed of sound within tissue.

Various embodiments described herein provide systems and methods for determining the location of a secondary intracorporeal device relative to a primary intracorporeal ultrasound imaging probe, where the primary intracorporeal ultrasound imaging probe is configured to scan a three-dimensional volume. In contrast to the aforementioned methods of performing ultrasonic locating of devices, various embodiments of the present disclosure employ the three-dimensional A-scan vectors associated with a three-dimensional scanning primary intracorporeal ultrasound imaging probe for actively locating a secondary device having ultrasound beacon transducers provided thereon. Accordingly, various embodiments described herein employ the highly directional nature of ultrasonic scanning devices to identify, via scanning a three-dimensional volume, the location of a secondary device based on directional, line-of-sight communication, between the primary intracorporeal ultrasound imaging probe and one or more ultrasonic beacon transducers located on the secondary device. It will be understood that although many of the example embodiments described herein refer to the locating of a secondary device, the systems and methods used herein may be employed to locate multiple devices having ultrasonic beacon transducers thereon.

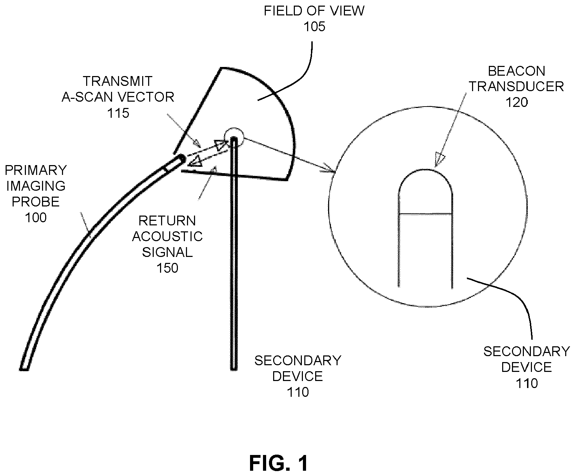

Referring now to FIG. 1, an illustration is provided of an example embodiment in which a primary intracorporeal imaging probe 100 is employed for scanning a three-dimensional region 105 (the imaging field of view), and also scanning the three-dimensional region to locate a secondary intracorporeal device 110 having an ultrasound beacon transducer 120. Primary intracorporeal imaging probe 100 includes an ultrasound imaging device that is configured to scan the three-dimensional region along a plurality of A-scan vectors 115. In the example embodiment shown in FIG. 1, ultrasound beacon transducer 120 of secondary intracorporeal device 110 is shown as a non-directional or omnidirectional ultrasound transducer, which has a wide angular acceptance range, although various additional and alternative beacon configurations and geometries are described below, provided that one or more ultrasonic beacon transducers are provided having a combined broad angular response. As noted above, unlike known methods of locating intracorporeal devices, the present example embodiment employs a scanning intracorporeal ultrasound imaging device to locate, based on the intracorporeal scanning of ultrasound energy, a secondary intracorporeal device having an ultrasound beacon transducer attached or otherwise associated therewith.

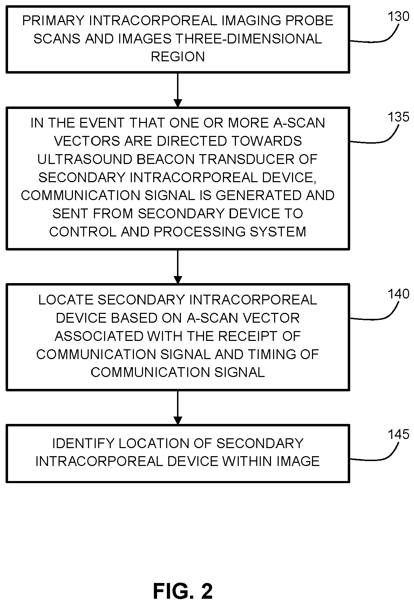

FIG. 2 provides a flow chart illustrating a method of locating secondary intracorporeal device 110 based on the scanning of primary intracorporeal imaging probe 100. At 130, primary intracorporeal imaging probe 100 scans the three-dimensional region 105, emitting ultrasound imaging energy, and receiving passively backscattered ultrasound imaging energy from material (e.g. tissue) within the three-dimensional imaging region. In the event that one or more A-scan vectors are directed towards ultrasound beacon transducer 120 of secondary intracorporeal device 110, as shown at 135, a communication signal is generated, and sent from the secondary device 110 to a control and processing system (which is interfaced with at least the primary intracorporeal imaging probe 100). At 140, the A-scan vector associated with the receipt of the communication signal, and the timing of the communication signal, is employed to locate ultrasound beacon transducer 120, and thereby locate secondary device 110. The position of secondary intracorporeal device 110 within the three-dimensional region 105 may then be determined, as shown at 145, and may optionally be shown in one or more images obtained via primary intracorporeal ultrasound imaging probe 100.

Such an active three-dimensional scanning approach provides improved locating performance over passive approaches involving imaging alone. Passive detection relies solely on the transmitted imaging beam to reflect off of the secondary intracorporeal device and to be sensed by the imaging device when in receiving mode. This limited interaction of the ultrasound signal, between the primary and secondary intracorporeal devices can make the reliable estimation of the position of the secondary intracorporeal device challenging relative to the surrounding tissue and relative to the imaging probe itself using passive imaging methods.

For example, when using a passive imaging approach, strong reflections from components and surfaces of the secondary device can lead to bright reflections, which may help to highlight the object within the image, but with reduced spatial resolution. In addition, if several surfaces of the secondary intracorporeal device are significantly parallel to the primary ultrasound imaging beam, then it may be problematic to determine the edge of the device, since the path of the reflected ultrasound imaging signal will mostly be directed away from the imaging transducer. The intensity of the acoustic echo received by the primary device may be quite small and blend in with the surrounding tissue or be perceived as noise. Also, the interaction of the acoustic imaging beam with the device and surrounding environment can disturb the direction of the acoustic energy and distort the representation of tissue around the secondary device. These may lead to artifacts known as shadowing, ghosting and time of flight distortions, to name a few such artifacts. Accordingly, when employing such passive imaging-based location approaches, depending on the extent to which a secondary device falls within the field of view, it may be challenging to satisfactorily determine the actual position and orientation of the device as required for its intended use and thus be challenging to adequately predict how the secondary device will interact with the surrounding tissue.

The systems and methods described herein, which generally relate to the use of a primary intracorporeal ultrasound imaging device for the active detection of an ultrasound beacon transducer on a secondary intracorporeal device based on the scanning of ultrasound energy, may be implemented according to a wide variety of scanning configurations, examples of which are described below.

In some example embodiments, the ultrasound imaging device may be configured to mechanically scan the three-dimensional imaging region. Some example implementations of a mechanically scanned ultrasonic imaging probe for scanning a three-dimensional imaging region within a subject is disclosed in U.S. Patent No. 8,214,010 (Courtney et al.). Mechanical scanning of an ultrasound imaging device may be achieved via a distal scanning element that is configured to scan a three-dimensional volume in which an ultrasonic transducer is mechanically scanned, or in which an acoustic beam deflector is mechanically scanned to scan an ultrasound beam emitted from an ultrasonic transducer. In some embodiments, the ultrasonic imaging device may be mounted on an imaging assembly that is attached or otherwise connected to a rotatable conduit or shaft (e.g. a torque cable) housed within a hollow sheath of the primary intracorporeal ultrasound imaging probe. In such a case, the scanning of the beam in the azimuthal direction (about an axis parallel to the rotation axis of the rotatable shaft) may be achieved due to rotation of the rotatable shaft, and scanning in the polar angle (subtended in a plane including the longitudinal axis of the rotatable shaft) may be achieved by another actuation mechanism, such as, but not limited to, an electromagnet, a steering cable mechanism, or a centripetal mechanism associated with the rotational velocity of the rotatable shaft, as described below.

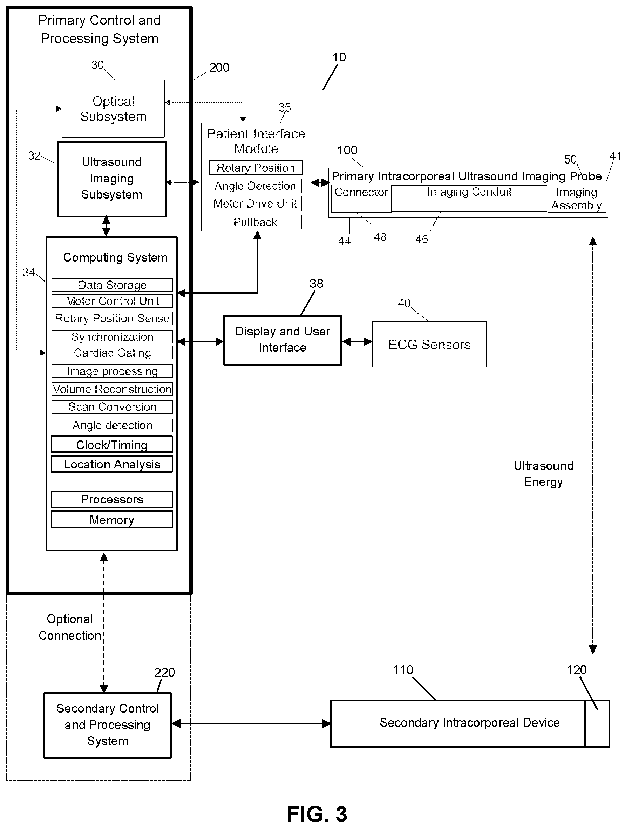

Referring now to FIG. 3, an example system 10 is shown for imaging a three-dimensional region with a primary intracorporeal ultrasound imaging probe 100 and locating the position of a secondary incorporeal device 110. Example system 10 includes primary intracorporeal ultrasound imaging probe 100, which connects via patient interface module 36 to primary control and processing system 200. Primary control and processing system 200 includes hardware to support one or more imaging modalities, such as ultrasound, optical coherence tomography, angioscopy, infrared imaging, near infrared imaging, Raman spectroscopy-based imaging, or fluorescence imaging.

Primary control and processing system 200 is employed to facilitate the coordinated activity of the many functional units of the system, and may contain some or all of the components shown in the Figure and listed herein. Computing system 34 includes one or more processors, memory, data storage, and processing modules (stored logic) for performing various methods described in the present disclosure. For example, computing system 34 includes clock/timing and location analysis processing modules for analyzing communication signals obtained from secondary intracorporeal device 110. The memory may store, for example, calibration data, such as calibration data pertaining to usable frequencies and configuration parameters for establishing the communication link.

Secondary intracorporeal device 110 is connected to secondary control system 220, which may include any of the components of control and processing system 200. As shown in the figure, secondary control and processing system 220 may be connected (directly or indirectly) to primary control and processing system 200. In some embodiments, secondary control and processing system 220 may be integrated directly with primary control and processing system 200 to form a single integrated control and processing system. These and other embodiments are described in further detail below.

An operator interacts with system 10 via display and/or user interface 38. System 10 may further include electrode sensors 40 to acquire electrocardiogram signals from the body of the patient being imaged.

Ultrasound subsystem 32 may include any or all of the following components: pulse generators, electronic filters, analog to digital converters, parallel processing arrays, envelope detectors, beam-forming circuitry, amplifiers including time gain compensation amplifiers and other components known to facilitate acoustic imaging techniques.

The example system shown in FIG. 3 illustrates the optional inclusion of one or more additional imaging modalities, in addition to ultrasound imaging. In one example implementation, the image obtained by the system may be obtained by an imaging modality other than ultrasound, while scanning ultrasound imaging energy is employed to locate the position of secondary probe 110 relative to the collected image data. Optional optical subsystem 30, if included in a particular implementation of an imaging system, may include any or all of the following components: interferometer components, one or more optical reference arms, optical multiplexors, optical demultiplexers, light sources, photodetectors, spectrometers, polarization filters, polarization controllers, timing circuitry, analog to digital converters, parallel processing arrays and other components known to facilitate any of the optical imaging techniques.

Example imaging probe 44 includes an imaging assembly 50, optional imaging conduit 46 along a substantial portion of its length, and connector 48 at its proximal end 47. Imaging assembly 50 is located near distal end 41 of imaging probe 44. Imaging assembly 50 generally refers to the components of the imaging probe 44 from which the signals (acoustic and optionally optical) are collected for the purposes of imaging a region that is proximate to imaging assembly 50. Imaging assembly 50 may house transducers for transmitting and/or receiving imaging energy. The emitter and receiver may be a single component, as is often the case with a piezoelectric transducer.

It is to be understood that patient interface module 36 and computing system 34 are but one example illustration of the selection and organization of hardware subsystems, and that many other implementations are possible. For example, patient interface module 36 may be housed with controller and processing systems 34 within processing and display system 49.

In the case of optical imaging, imaging assembly 50 typically contains the distal tip of a fiber optic, as well as a combination of optical components such as a lens (for instance, a ball lens or a GRIN lens). A mirror and/or prism may be included for use in beam delivery and/or collection. Optionally, there may be an optical detector, such as a CCD array, or an optical light source, such as one or more LEDs, incorporated directly in the imaging assembly that may obviate the need for one or more fiber optics in an optical imaging probe.

Imaging probe 44 may contain ports at one or more points along its length to facilitate flushing. Moreover, imaging assembly 50, connector 48 and/or imaging conduit 46 may be filled and / or surrounded with a fluid such as saline, and may be flushed.

Imaging conduit 46 includes at least one conductive wire (optionally two or more) that connect an emitter and/or receiver via connection to an adapter, herein referred to as patient interface module 36.

Patient interface module 36 facilitates transmission of signals within any fibers and/or wires to the appropriate image processing systems. It may contain a motor drive unit for imparting motion to the components of the imaging mechanism.

In many applications, it can be important to optimize the geometry of a minimally invasive probe so that it is as small as reasonably possible to achieve its desired purpose. Current intravascular ultrasound (IVUS) and intracardiac echocardiography (ICE) probes are approximately 0.9 to 4 mm in diameter and the smaller sizes of probes can be delivered more distally within the vascular tree of the coronary anatomy as the vessel caliber tapers down or as diseased vessels are stenosed. Furthermore, within the cardiac anatomy, smaller probes (such as those with a diameter less than about 4 mm) can be readily advanced across the atrial septum into the left atrium of the heart. Thus, smaller sizes generally allow for delivery of the device into a larger portion of the coronary or cardiac anatomy. It is therefore desirable for a probe and its components to be contained within a minimal outer diameter to enable imaging, such as using imaging performed with the scanning mechanisms described by Courtney et al. (US Issued Patent No. 8,214,010).

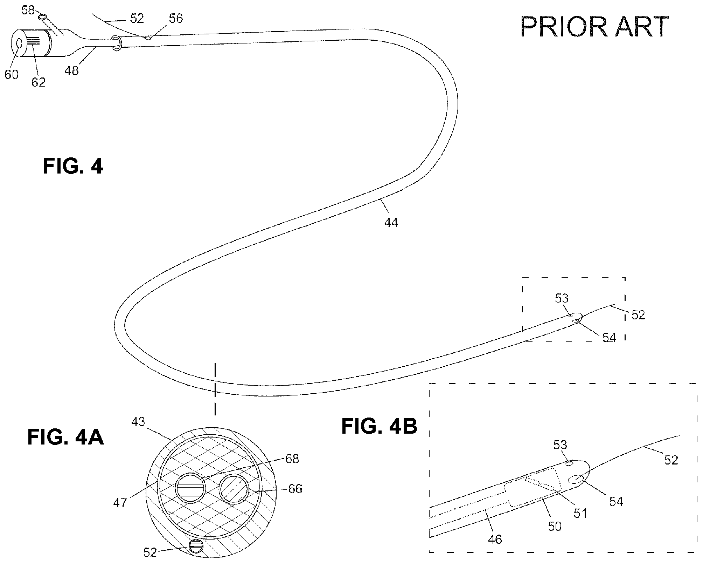

FIGS. 4, 4A, 4B and 5A-5K illustrate several example implementations of an incorporeal imaging probe that is configured to scan a three-dimensional volume, as disclosed in US Patent Publication No. 20080177138, titled "Scanning Mechanisms for Imaging Probe" and filed on January 22, 2008 and US Patent Publication No. 20090264768, titled "Scanning Mechanisms for Imaging Probe" and filed on March 27, 2009.

FIG. 4 is a perspective drawing of an example flexible catheter containing fiber optic 66 and co-axial electrical cable 68. The proximal connector contains fiber optic connection joint 60 that can be received by patient interface module 36 to optically couple imaging fiber optic 66 to primary control and processing system 200. Electrical connectors 62 allow one or more electrical conduits to be connected to the ultrasound circuitry and/or controller and processing systems. In applications in which the imaging conduit rotates around its longitudinal axis, there may be a need to couple the rotating components of the imaging fiber optic with a relatively stationary fiber optic that connects primary control and processing system 200. This coupling can be achieved with the use of a fiber optic rotary joint incorporated either as part of the proximal connector of imaging probe 48 or as part of patient interface module 36. Similarly, there may need to be a mechanism for coupling the rotating components of the electrical system with relatively stationary electrical components that connect to primary control and processing system 200. This can be achieved through the use of one or more electrical slip rings or slip ring channels.

FIG. 4A shows a cross sectional view of the middle section of the catheter shown in FIG. 4 taken along the dotted vertical line. The cross section shows the optional fiber optic 66, optional guidewire 52, imaging conduit lumen 47, external sheath 43, which is a hollow, flexible elongate shaft made of physiologically compatible material and having a diameter suitable to permit insertion of the hollow elongate shaft into bodily lumens and cavities, and co-axial wiring 68. The expanded detailed view of the end of the imaging probe 44 in FIG. 4B shows the imaging assembly 50 which optionally includes a tiltable member 51, distal end of the optional guidewire 52 extended beyond the end of the external sheath 43 and a flush port 53 near the end of the sheath 43. In FIG. 4, the proximal end of the imaging probe 44 includes an optional guidewire port 56 into which the guidewire 52 is inserted and the connector assembly 48 includes a flush port 58 and electrical contacts 62 along with the connector body. An optional guidewire port 54 is seen in FIG. 4B.

FIGS. 5A-D show an example imaging probe that employs a tiltable member for scanning an imaging beam. FIG. 5A shows a perspective cutaway drawing of the distal region of an imaging probe 44 that relies on centripetal force to generate the change in tilt angle of the tiltable member 51. The imaging probe 44, which includes a sheath 43 for isolation from bodily fluids and cavities, includes tiltable member 51, which may be housed within an imaging assembly, as shown in FIG. 4B.

Tiltable member 51 is mounted on pins 102, about which tiltable member 51 is able to pivot and is bias towards its starting position with the use of a restoring force. As imaging conduit and assembly (not shown) are rotated about longitudinal axis 59 at a slow rate (indicated by arcing hatched arrow 61), the angle α subtended between longitudinal axis 59 and tiltable member 51 is relatively small. A cutaway perspective cross-sectional view of FIG. 5A is shown in FIG. 5B. FIG. 5C shows a similar drawing of the distal region of imaging probe 44 as shown in FIG. 5A, except with imaging conduit 46 being rotated at a faster rate (indicated by arcing hatched arrow 63) than in FIG. 5A. Centripetal force causes tiltable member 51 to tilt such that there is an increase in the angle α subtended between the longitudinal axis of the catheter and the tiltable member 51. FIG. 5D is a cutaway perspective cross-sectional view from FIG. 5C.

FIG. 5E shows a perspective cutaway drawing of the distal region of a related imaging probe 44 that relies on the use of dynamically controlled magnetic fields to change the deflection angle of tiltable member 51. Imaging probe 44, which may include a sheath 43 for some degree of isolation from bodily fluids and cavities, includes tiltable member 51 comprising part of the imaging assembly 50. Tiltable member 51 is mounted on pins 102, about which the tiltable member 51 is free to pivot. Mounted on the tiltable member 51 is a magnetically influenced element 109 that can be either attracted or repulsed by a magnetic field. For example, it may be a ferromagnetic component, or a permanent magnetic component. Element 109 may integrally be part of tiltable member 51, such as if all or a portion of element 109 is made of either a ferromagnetic or magnetic substrate. An electromagnetic component 107 is also placed at a position separate from the tiltable member 51. The electromagnetic component can be controlled to produce attractive or repulsive forces relative to magnetically influenced component 109. In so doing, the angle α subtended between the longitudinal axis 59 of the catheter and the tiltable member can be adjusted as desired. Furthermore, similar imaging probes may be conceived that involve interchanging the position of the electromagnetic component 107 and magnetically influenced component 109, or using two electromagnets instead of an electromagnet and a magnetically influenced component. A cutaway perspective cross-sectional view of FIG. 5E is shown in FIG. 5F.

FIG. 5G shows a similar drawing of the distal region of imaging probe 44 as shown in FIG. 5E, except with a repulsive sequence applied to electromagnet 107 such that the angle α subtended by tiltable member 51 is increased. FIG. 5H is a cutaway perspective cross-sectional view from FIG. 5G.

Tiltable member 51 may be an ultrasonic transducer, such as an ultrasound transducer used for producing B-scan ultrasound images. Another embodiment includes an ultrasound transducer mounted on a tiltable member.

FIG. 5I shows an example of a potential scanning pattern for generating ultrasound images. In this case, the tiltable member is an ultrasound imaging transducer 101. As imaging conduit and assembly (not shown) are rotated at a constant rate, an image is generated along a surface that approximates a cone. As the rate of rotation is changed, centripetal force causes the angle subtended between the longitudinal axis of the catheter and ultrasound imaging transducer 101 to change. This can result in a path that approximates a series of concentric imaging cones 118 for different rotational speeds. The angle subtended between the longitudinal axis of the catheter and an axis normal to ultrasonic imaging transducer 101 will be referred to as the "imaging angle". In this case, the transducer begins with a relatively small imaging angle θ1 implying a fast rate of rotational speed. As the rotational speed is reduced, the imaging angle is increased to θ2.

In some embodiments, a mechanism may be provided for detecting the tilt angle of the tiltable member. A number of example implementations are described in PCT Patent Application No. PCT/CA2012/050057, titled "ULTRASONIC PROBE WITH ULTRASONIC TRANSDUCERS ADDRESSABLE ON COMMON ELECTRICAL CHANNEL". As shown in FIG. 5J, the imaging angle may be employed for feedback in a control system. A desired angle 194 and the measured angle 192 are provided as inputs to controller 196, and the output of controller 196 is provided to angle control mechanism 190. A variety of control methods and algorithms known in the art may be employed, including, but not limited to, PID and fuzzy logic controllers.

In order to cause the imaging angle to return to a stable position in the absence of rotation, a restoring mechanism can be used as shown in FIG. 5K Here, the primary movable member 101 is connected to a secondary movable member 114 using a mechanical coupler 176, allowing the two members 101 and 114 to move synchronously. All components are housed within a shell 178. One or more springs 182 are connected between the movable member 101 and the shell 178. The springs may be torsion springs, linear springs, or a cantilever spring. The movable members 101 and 114 are pivotally supported by around pins 111 and 113 respectively. This spring 182 provides a force to restore the member 101 to the side viewing position in the absence of adequate rotational force to overcome the restoring force provided by spring 182. In addition to adding a mechanical restoring force, the torsional springs may also be formed, at least in part, from an electrically conductive material, such as stainless steel, beryllium copper, copper, silver, titanium, gold, platinum, palladium, rhenium, tungsten, nickel, cobalt, alloys that include one or more of these metals and many other metals and their alloys can be used to provide electrical connections. Here, spring 182 is in electrical communication with conductor 300. Conductor 301 makes a similar connection to the opposite side of movable member 101 (not shown).

In another example embodiment, the primary intracorporeal imaging ultrasound probe may employ a phased array of transducer elements. In one example implementation, a linear phased array of ultrasonic transducer elements may be provided on a rotating shaft or conduit housed within a hollow sheath of the primary intracorporeal ultrasound imaging probe, where the linear array is arranged along a direction parallel to the longitudinal axis of the probe. The linear array may be employed to scan a two-dimensional region within a plane including the longitudinal axis of the probe, which may be rotated via the rotatable shaft to achieve three-dimensional scanning.

In various embodiments, the location of the secondary intracorporeal device 110 is determined based on the detection, by one or more ultrasound beacon transducers 120, of ultrasound energy emitted along a particular A-scan vector, originating from the primary intracorporeal ultrasonic imaging probe 100, and the subsequent transmission of a communication signal between secondary intracorporeal device 110, and primary control and processing system 100. As described in various embodiments below, the ultrasound energy transmitted, in a scanning configuration along multiple A-scan vectors, from primary intracorporeal ultrasonic imaging probe 100 to secondary intracorporeal device 110, may reside within the imaging bandwidth of primary intracorporeal ultrasonic imaging probe 100 (i.e. within the imaging band), or may reside in a separate communication band that is distinct and separated, in the frequency domain, from the imaging band. Also, according to various example embodiments, the transmission of the communication signal from secondary intracorporeal device 110 to primary control and processing system 200 may be achieved according to an acoustic link between the ultrasonic beacon transducer of secondary intracorporeal device 110 and primary intracorporeal ultrasonic imaging probe 100 (within the imaging band, or within a separated communication band, as noted above), or via a non-acoustic transmission between secondary intracorporeal device 110 and primary and control system 200. Examples of these different methods and systems are described in further detail below.

As noted above, the one or more ultrasound beacon transducers 120 on secondary intracorporeal device 110 provide for the active detection of incoming ultrasound energy emitted, along a given A-scan vector, by the ultrasonic imaging device of the primary intracorporeal ultrasonic imaging probe 100. The fact that a given ultrasonic beacon transducer 120 attached to secondary intracorporeal device 110 detects an acoustic signal means that the position of the ultrasonic beacon transducer 120 is in the path of the ultrasound beam originating from primary intracorporeal ultrasound imaging probe 100, and thus defines an event in time that can be correlated to the spatial vector linking primary intracorporeal ultrasound imaging probe 100 and secondary intracorporeal device 110, and can therefore be associated with the known direction of the ultrasound vector that originated from primary intracorporeal ultrasound imaging probe 100.

As noted above, the one or more ultrasound beacon transducers 120 on a secondary intracorporeal device 110 provide for the active detection of incoming emitted ultrasound energy, along a given A-scan vector, generated by the ultrasonic imaging device of the primary intracorporeal ultrasonic imaging probe 100. According to one example implementation involving the use of a secondary system, each of the beacon transducers may have an associated ID code stored in memory on the secondary process and control system. The ID code may contain calibrated data defining the response characteristics (including the position of the beacon on the secondary device and latencies) of the beacon transducer in question. The fact that a given ultrasonic beacon transducer 120 attached to the secondary intracorporeal device 110 detects an acoustic signal means that the position of the ultrasonic beacon transducer 120 is in the path of the ultrasound beam originating from the primary intracorporeal ultrasound imaging probe 100, and thus defines a communication event in time that can be correlated to the spatial vector linking the primary intracorporeal ultrasound imaging probe 100 and the secondary intracorporeal device 110, and can therefore be associated with the known direction of the ultrasound vector that originated from primary intracorporeal ultrasound imaging probe 100.

Determining the 1-way time of flight between the imaging assembly of the primary device and the beacon transducer of the secondary device can be achieved in several ways. For example, if the transmitted imaging energy is used to establish an active communication link leading to a communication signal then the timing can be monitored by keeping track of the time of the initial imaging trigger (from the primary control and processing system) and the arrival time of the received ultrasound energy from a beacon transducer. According to the present example, this arrival time would first be determined on the secondary control and processing System and can be communicated through an external connection to the primary control and processing system. As noted elsewhere, the external connection may be, for example, one of an electrical connection, a wireless connection, and an optical connection. The difference in timing would represent the 1-way ultrasound propagation time plus the electrical response time. The electrical response time of the control and processing systems will be on a time scale that is much shorter than the acoustic time of flight.

The secondary control and processing system will also determine and store at least one acoustic parameter of the received ultrasound energy within the ultrasound bandwidth of the beacon transducer that received said ultrasound energy (for example: peak intensity, pulse duration of received waveform, operational frequency, bandwidth of waveform spectrum, the intensity of amplitude modulation) to identify the nature of said energy that originated from the Primary probe. The clocks between the Primary and Secondary Control and Processing systems would be synchronized. For example: the Primary system could simultaneously send a timing synchronization signal to the secondary system when it sends the imaging trigger to the primary ultrasound imaging subsystem such that the secondary system can independently determine the time difference between the triggering of the image signal and the receipt of the ultrasound energy from the beacon transducer.

Any latency in the delay time between sending the imaging energy relative to the initial imaging trigger could be stored in the memory of the primary control and processing system and is a value that can be used as a timing correction factor. Any latency in the delay between the reception of received ultrasound energy from a beacon transducer and the actual time stamping of that signal relative to a timing clock signal in the secondary control and processing system can be stored as calibration data in said secondary system and is a value that can be used as a timing correction factor.

The primary system, which already builds a header of information associated with each A-scan vector of imaging data, for the reconstruction of 2D and 3D images, could add additional information to said header in relation to the active communication link. Such parameters would include, but is not limited to: bidirectional active ultrasound link received (yes or no), 1-way active ultrasound link received (yes or no), time stamp of the imaging trigger, time stamp of the received ultrasound energy, the beacon transducer ID code, timing correction factors, all determined acoustic parameters of the received ultrasound energy within the ultrasound bandwidth of the beacon transducer used to identify the nature of the energy that originated from the Primary probe, etc...

If the above example is modified such that the transmitted ultrasound energy, from the primary probe, intended to initiate an active communication link with a beacon transducer is a waveform that is more narrow band than the imaging waveform, and the communication trigger is initiated with a time lag after the imaging trigger, then the timing of the triggering of both the imaging waveform and communication waveform can be monitored by keeping track of both triggers used and the arrival time of the received ultrasound energy from a beacon transducer can be communicated through the external connection between the primary and secondary control and processing systems. As identified above, any latency between the transmission of either the imaging energy or the communication energy relative to their respective triggers, in the primary system, can be stored in the memory of the primary control and processing system and is a value that can be used as a timing correction factor. Any latency in the processing of the received beacon signal that would at least be dependent on the fact that the imaging and the communication waveforms may be in different frequency bands could also be calibrated and stored in the memory of the secondary control and processing system. Similar to above, at least one acoustic parameter of the received ultrasound energy is determined (for example: peak intensity, pulse duration of received waveform, operational frequency, bandwidth of waveform spectrum, or the intensity of amplitude modulation) to identify the nature of said energy that originated from the primary probe and stored in memory of the secondary control and processing system and transmitted to the primary processing and control system via the external link.

An additional example is provided here for narrowband communication signals. According to this additional example, if a specific feature of the received communication waveform is used to determine the arrival time of the received ultrasonic energy by the beacon transducer, for example: the sixth maximum of a 10 cycle tone burst, then a correction factor equivalent to the time of propagation between the identified feature and the start of the communication waveform, which would be a time latency that is based on the speed of sound and would be 'long' relative to the electrical latencies, can be corrected for. The knowledge of the communication signal waveform would be required to perform this correction and this information can be stored in the memory of the primary control and processing system. All pertinent information related to the transmitted imaging and communication energies and the receipt of ultrasound energies by a beacon transducer (either a broadband imaging waveform, or a communication waveform), can be included in the header of the A-scan vector generated by the primary ultrasound imaging subsystem. This information would be used during the reconstruction of a 2D or 3D image.

In the case where multiple beacon transducers are receiving acoustic energy intended for communication, then each electrical circuit associated with each beacon would each have their own control and processing latencies calibrated and stored in memory of the secondary control and processing system and could be transmitted to the Primary system via the external link. The positions of each of the beacons (longitudinally and radially within the transverse plane of the secondary device) would also be known as well as the acoustic response transmitted from each of the beacon transducers. This information would also be stored in memory in the secondary system and could be transmitted to the primary system via the external link

In the case of an active ultrasound communication link between the primary probe and the secondary device, the entire secondary system response time from the receipt of received communication energy by a beacon transducer, to the determination that an acoustic response from the secondary control and processing system, is required and to the latency between sending a trigger to actuate the same beacon transducer and the actual transmission of the return communication energy can be measured and stored as a calibration parameter in the secondary control and processing system. The combined latency can be communicated back to the primary control and processing system via the external link between the two systems, to be used as a correction factor by the primary system to adjust or calibrate the timings.

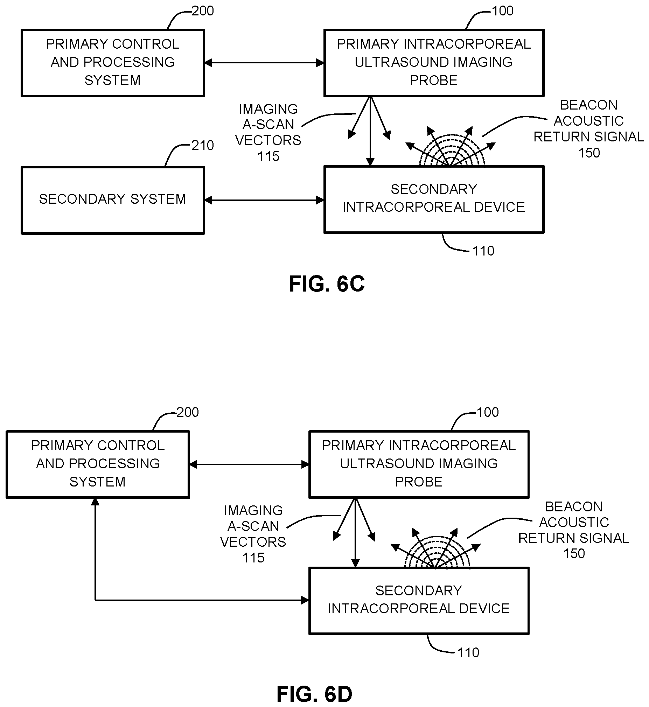

The received ultrasound communication energies detected by the imaging assembly of the primary imaging probe can be processed by the primary control and processing system in manners that are consistent with all example methods presented for the secondary control and processing systems and similar associated latencies of the primary control and processing system can be stored in the memory of the primary system. All received ultrasound communication energies by the primary probe represents a bidirectional ultrasound communication link that may or may not have additional and complimentary information communicated via an external link (FIG. 6C and 6D respectively). The associated received processing parameters and stored latencies can also be included in the imaging A-scan vector header as bidirectional communication data that can be used to estimate the 2-way time of flight distance between the primary imaging assembly and the beacon transducer responsible for transmitting the returned communication energy. If there is no external link between the primary and secondary systems, then any latencies of the secondary system can still be taken into account (for example: latencies can be entered by the operator of the system from accompanying documents or calibration data associated with the secondary intracorporeal device). If an external link exists, then both 1-way active communication and bidirectional active communication information can be used in tandem to reconfirm the existence of an active communication link associated with any particular beacon transducer of the secondary device and aid in improving the estimate of the position and orientation of the secondary device.

An active link can be achieved using imaging and communication energy transmitted along the imaging A-scan only or with additional communication energies that are transmitted along known directions relative to the imaging A-scan vector using a grating transducer as part of the imaging assembly in the primary imaging probe (e.g. as shown in FIG. 28). This means that multiple bidirectional communication link events may be received by the primary imaging probe along a single A-scan vector. This also means that the direction (orientation) of the receipt of a single active communication event may not be independently resolvable. By scanning a 3D volume there will be multiple A-scans where each beacon can establish a bidirectional communication link or a 1-way communication link. During 3D reconstruction, the ambiguities of a single event can be eliminated by correlating the active communication link data in the header (Beacon transducer ID code, time of flight distance data, Imaging and communication A-scan vector directions,...) for each A-scan vector that identified a communication link event. For example, in reference to FIG. 28, where the -1 and 1 lobes of the linear diffraction grating are at an angle of 24.6 degrees relative to the Imaging A-scan Vector and the secondary device has a single omnidirectional beacon transducer at the distal tip of the device (as shown in FIG. 1). If a 3D scan is obtained with the transducer tilt angle ranging from 0 degrees to 90 degrees and the A-scan header information identifies communication events at transducer tilt angles of 15.4 and 40 degrees respectively, then the beacon transducer placed on the tip of the secondary device will be located along the 40 degree A-scan vector within the 3D scanned imaging volume at a distance that is equal to the time of flight data stored in the respective headers. The - 1 and 0 lobes provided the active communication links at 15.4 and 40 degrees respectively.

With the use of more than one beacon transducer on a secondary device, the orientation of the secondary device can be estimated by trigonometry and comparing the known relative positions of the beacons on the secondary device to the distances determined by the primary control and processing system (using the header information from each imaging A-scan that contains valid active communication data for both 1-way communication links and bidirectional communication links).

Using all A-scan vector headers that contain a specific Beacon ID code, it is possible to determine the beacon position in space relative to the imaging assembly of the primary probe. This process can be repeated for each Beacon ID code contained within the 3D scan header data.

Knowing the relative placement of the beacon transducer on the secondary device, the position and orientation of the secondary device can be determined.

It is further noted that the beam profile of any primary transducer and of any beacon transducer is not infinitesimally small in cross section. This means that if an active communication link is detected, then in reality, a cluster of consecutive A-scans will generate a similar set of timing and latency parameters to be included in the header of each A-scan transmitted by the imaging assembly of the primary device. The use of a threshold (for example: the peak intensity of the received communication energy by the imaging assembly or the beacon transducer) can be used to reduce the number of consecutive a-scans where the event of an active communication link is detected. For a set of consecutive A-scans that is larger than one (1), there will be slight differences in the recorded time of flight between the transducer devices involved in the link, however each A-scan of the cluster of A-scans could be used to average the estimate of the orientation of the beacon transducers in 3D space when estimating the orientation of the secondary device.

In the case where the beacon detecting a communication signal is being operated in an echo backscatter mode (see FIG. 8), there would be no latency to be tracked by the secondary control and processing system as the return communication signal would simply be a modulation of the reflected signal.

In the case where there is more than one beacon transducer associated with the secondary device, and the beacons themselves are used to self-triangulate (refer to FIG. 11) by means of the second control and processing system alone, then this information can be communicated via the external link to the primary control and processing system and this information can be used to further refine the prediction of the orientation and position of the secondary device relative to the primary probe.

In some embodiments, the ultrasound energy that is detected by one or more ultrasonic beacon transducers 120 is the imaging ultrasound energy emitted by primary intracorporeal ultrasound imaging probe 100 when scanning the three dimensional volume. An example of such an embodiment is shown in FIG. 6A, in which the communication signal is provided through a non-acoustic separate signal channel. For example, the non-acoustic channel may be an electrical communication path, such as one or more electrical conductors that carry an analog or digital signals, a wireless signal channel, an optical signal channel (such as an infrared line of sight channel) or one or more fiber optics. FIG. 6B illustrates a similar embodiment in which the non-acoustic signal channel is provided from secondary intracorporeal device 110 to primary control and processing system 200.

In one example embodiment, the communication signal may comprise analog radiofrequency data. In another example embodiment, the communication signal may additionally or alternatively comprise triggering and/or timing signals.

It is noted that the many of the example embodiments provided herein employ the active communication involving only the one-way transmission of any given ultrasonic energy, which is advantageous over passive approaches involving two-way acoustic propagation, because the one-way transmission path involves a shorter path length. Accordingly, the detected acoustic signal being used for localization will be of stronger intensity, and the beam profile will be of smaller cross section.

Furthermore, the initial detection of ultrasonic energy by the secondary intracorporeal device occurs in a time interval that is approximately equivalent to the propagation of the ultrasound from device to device and is dependent on the speed of sound in the propagating medium, as well as processing time to interpret the received signals, which is in turn dependent on computer processing (potentially a much shorter time scale). Accordingly, for an active approach, the interpretation of the received acoustic signal is performed electronically after 1-way acoustic propagation and can be relayed back to the primary device system via analog or digital communication. In contrast, for a passive technique, the acoustic time is dependent on two-way propagation of the acoustic energy before any processing of received acoustic data can take place.

FIG. 6C illustrates an example implementation in which the communication signal is provided as a return acoustic communication signal that is emitted from ultrasound beacon transducer 120 of secondary intracorporeal device 110 and detected by primary intracorporeal ultrasonic imaging probe 100. In one embodiment, the acoustic communication signal is emitted within the imaging band associated with the ultrasound imaging device within primary intracorporeal ultrasound imaging probe 100. In such a case, the return acoustic communication signal emitted from the ultrasound beacon transducer is provided such that it is differentiable from passive ultrasound imaging energy received by primary intracorporeal ultrasound imaging probe 100. Furthermore, the acoustic communication signals from a given ultrasound beacon transducer is differentiable from other active acoustic responses originating from other ultrasound beacon transducers that may also be attached to the same secondary intracorporeal device, but fixed at a different location or attached to other secondary intracorporeal devices that may also be similarly fixed with ultrasound beacon transducers. According to some non-limiting example implementations, the acoustic communication signal may be differentiated based on one or more of frequency, intensity, tone bursts with or without modulation, chirped waveforms, and other formed of coded signal transmission.

Referring again to FIG. 6C, as shown in the illustrated example embodiment, secondary intracorporeal device 110 may be independently controlled by a secondary control and processing system 210 that need not be connected to primary control and processing system 200. Alternatively, as shown in FIG. 6D, secondary intracorporeal device 110 may be directly controlled by primary control and processing system 200

FIG. 7 illustrates the relevant timescales that pertain to the systems and methods described herein. The above examples provided to explain how ultrasonic communication events in time can be correlated to the spatial imaging and communication A-scan vectors linking primary intracorporeal ultrasound imaging probe 100 and secondary intracorporeal device 110, and can therefore be associated with the known direction of the ultrasound vectors that originated from primary intracorporeal ultrasound imaging probe 100 and make use of the different time scales described in this figure.

Referring now to FIG. 8, an example embodiment is illustrated in which one or more of ultrasound beacon transducers 120 of secondary intracorporeal device 110 is configured in an echo-backscatter mode. Methods of performing echo-backscatter mode for modulating reflected ultrasound energy from an ultrasound transducer are provided, for example, in Mazzilli, F. et al., In-Vitro Platform to study Ultrasound as Source for Wireless Energy Transfer and Communication for Implanted Medical Devices, 32nd Annual International Conference of the IEEE EMBS, Buenos Aires, 2010, and in Mari, J.M. et al., Detection of Deeply Implanted Impedance-Switching Devices Using Ultrasound Doppler, IEEE Transactions on Ultrasonics, Ferroelectrics, and Frequency Control, vol. 60, no. 6, June 2013.

It is noted that while the embodiments illustrated in FIGS. 6A-6D may be performed in which the ultrasonic beacon transducers 120 detect, and in some embodiments, emit ultrasonic energy within the imaging band, other example embodiments may involve out-of-band detection and/or emission of ultrasound energy, employing a distinct communication band for location sensing. Examples of such embodiments, and examples of ultrasound imaging transducers suitable for dual-band operation, are described in detail below.

It will be understood that the one or more ultrasonic beacon transducers 120 associated with secondary intracorporeal device 110 may be provided according to a wide range of geometries and configurations. As shown in FIGS. 1 and 3, secondary intracorporeal device 110 includes one or more ultrasonic beacon transducers 120. Piezoelectric sensors, capacitive micromachining ultrasound transducers (CMUTs), and optical etalon sensors are a few exemplary sensors that can be fixed to, incorporated into, or coupled with a secondary device. The location, orientation, and directivity pattern of the sensors integrated into the secondary device all play a role in the ability of the secondary device to receive the acoustic signal, interpret the parameters of the signal and respond to the received signal. Generally, the set of ultrasonic beacon transducers provided on secondary intracorporeal device 120 are provided such that their combined angular beamwidth is broad, such that incident ultrasound energy from primary intracorporeal ultrasonic imaging probe 100 can be detected over a wide range of positions and orientations of secondary intracorporeal device 120 relative to primary intracorporeal ultrasonic imaging probe 100.

As noted above, it is preferable that the one or more of ultrasonic beacon transducers 120 fixed to secondary intracorporeal device 110 are sensitive to incident ultrasonic energy from as many directions as possible as the relative position and orientation of secondary intracorporeal device 110 with respect to the primary intracorporeal ultrasonic imaging probe 100 can vary substantially. On each individual secondary intracorporeal device, this could be achieved with a single non-directional sensor that may be dome shaped (see FIG. 1) or that may approximate a point receiver.

According to one example, a secondary intracorporeal device with a single dome shaped aperture transducer made from polymer PVDF, or copolymer, and mounted at the distal tip of the secondary device can provide a broad range of frequency sensitivity over a wide range receiving angles, as shown in FIG. 1. The smaller the size of the dome shape the better the ability to determine the relative position of the sensor with respect to the transmitting device as fewer A-scan vectors will be detected by the ultrasonic beacon transducer 120 on the secondary intracorporeal device 110.

In another example embodiment, a plurality of ultrasound beacon transducers 120 may be provided, where each sensor may have a different degree of directivity ranging from non-directional to highly directional, whereby the plurality of ultrasound beacon transducers are oriented in a wide range of directions. Examples of such embodiments are shown in FIGS. 9, 10 and 11.

In FIG. 9, multiple ultrasound beacon transducers 121 are shown provided on different portions of secondary device 110. In FIG. 10, multiple ultrasound beacon transducers 122 are shown provided on different portions of trifurcated distal segments of secondary device 110. Generally, the larger the number of ultrasonic beacon transducers that are distributed along the body of the secondary device, the better the ability to determine the orientation of the secondary intracorporeal device 110 relative to primary intracorporeal ultrasonic imaging probe 100.

In cases in which each ultrasonic beacon transducer is configured to emit ultrasonic signals in response to having received ultrasonic energy, it is advantageous (but not necessary) if the emitted ultrasonic energy from each ultrasonic beacon transducer is unique, as this would allow for the primary control and processing system 200 to differentiate between the plurality of received ultrasonic signals that are to be decoupled from the normally expected received signals along any given A-scan vector based on differentiating the nature of the received communication A-scan vectors alone. One or more ultrasonic beacon transducers 120 may take on different sizes and geometries to be sensitive to different frequency bands of acoustic signals or may be highly directed in their sensitivity to be sensitive to acoustic energy coming from a specific location.

In one example embodiment, a series of transducers positioned along the shaft of the secondary device may also provide means of determining the orientation of the secondary device due to shadowing from the acoustic beam caused by the main body of the secondary device itself, as illustrated in FIG. 11, since a shadowed ultrasonic beacon transducer would not detect a received ultrasound signal and would not actively transmit a response and thus its Beacon ID code would not be detected by the primary device. For example, active localization can be applied to the known position and orientation and relative spacing between a plurality of ultrasonic beacon transducers positioned on an undeflected secondary intracorporeal device. As the secondary intracorporeal device is deflected/manipulated during normal use, the system of sensors themselves can be used to self-triangulate to provide an estimate of the complex orientation of the deflected or of the manipulated device. In other words, the spatial distribution of ultrasonic beacon transducers on the secondary intracorporeal device can be used to augment the users understanding of the orientation and position of the secondary intracorporeal device.

The larger the number of ultrasonic beacon transducers and secondary devices, the greater the opportunity to triangulate between a set of acoustic transducers using a broader range of acoustic parameters (such as frequency, intensity, pulse bandwidth variations, as well as amplitude and frequency modulation, to name a few).

It is further noted that the type of ultrasonic beacon transducer, frequency bandwidth, size, orientation and receive directivity profile all play a role in the ability of the secondary intracorporeal device to receive and detect ultrasound energy from the primary intracorporeal ultrasound imaging probe, and to interpret the parameters of the signal and respond to the received signal (either through a returned acoustic signal or via an electrical or optical communication path including either analog or digital signal communication).

As noted above, the embodiments described herein are not limited to the sensing of a single secondary intracorporeal device, and may be extended or adapted for the detection of, and optionally mutual triangulation among, multiple secondary intracorporeal devices.

All permutations of active communication between the primary and secondary devices can be extended to include a plurality of devices which can in turn be considered as a system, or a network of devices, whereby devices may act as primary or secondary devices in relation to each other. Some of the devices may be located inside or outside of the body. Devices that are not within the field of view of the primary device are tertiary devices (See FIGS. 11 and 12) and may operate in a manner as shown in the US Patent No. 4,249,539. A benefit of incorporating a tertiary device in the system and methods disclosed is that the tertiary device could have fiducials on them that could be tracked by a tracking system, thereby allowing one to register the imaging probe, the image from the imaging probe, and the position of the secondary device within a global reference frame.

In one example embodiment, the nature of the relative motion between the secondary intracorporeal device and the primary intracorporeal imaging probe can be determined. For example: The primary control and processing system can compare, for a given temporal period (or a set of given temporal periods), the imaging signals from a local positional cluster of A-scans with the communication signals of the same cluster of A-scans, and if relative motion is determined between the intracorporeal devices using communication information (either by a change in time of flight to a given beacon transducer, or a change in A-scan clusters where communication links are created as the primary imaging assembly is rotated through several 360 rotations) then this can be compared to the relative motion of tissue as determined using imaging information. Depending on the degree of correlation between the perceived motions from both sets of ultrasound bands of energy the primary control and processing system can determine if the relative motion was due to the primary device being rotated or translated, or if it was the secondary device that moved. If the position of the secondary device has shifted relative to the surrounding tissue, or more subtly, if the cluster of A-scans (within the relative reference frame of the Primary imaging probe) that interact with the beacon transducers changes then this can be compared to the slower temporal motion associated with the background imaged tissue and any of its motion due to for example: cardiac cycles or patient twitches as indicated in FIG. 7.

Although many of the figures and examples provided herein show the secondary device as a probe-type device, it will be understood that the secondary device can take on a wide variety of forms. For example, a non-limiting list of examples of secondary devices includes the following: Brockenbrough needles, guidewires, biopsy probes, other ultrasound catheters (both for imaging and therapeutic applications), RF ablation tools, pacing catheters (both temporary and implantable), ventricular assist device cannulae, Swan-Ganz catheters, thermo dilution catheters, pressure sensing catheters, balloon catheters, stents, drug and nutrient delivery catheters (IV therapy and targeted delivery), drainage catheters, , optical probes(Endoscopic, OCT or other), rotational endarterectomy devices, embolic protection devices, catheters containing devices to be deployed including left atrial appendage occluders, transcutaneous aortic valve replacements, mitral repair devices, septal occluders, or any other diagnostic or imaging minimally invasive device that may be used in conjunction with the primary intracorporeal ultrasound imaging probe to complete a medical procedure and can also include implantable devices like pacemakers.

The following examples are presented to enable those skilled in the art to understand and to practice embodiments of the present disclosure. They should not be considered as a limitation on the scope of the disclosure, but merely as being illustrative and representative thereof.

The following section describes an example implementation of a method of performing intracorporeal imaging while locating a secondary intracorporeal device via ultrasound scanning. Primary intracorporeal ultrasonic probe 100 is placed into the patient's body and navigated to an organ or region of interest (for example, the heart). As described above, primary intracorporeal ultrasonic imaging probe 100 is electrically connected to control and processing system 200 that controls the 2D and 3D scanning of the primary intracorporeal ultrasonic probe 100 and the display of the received ultrasonic echo energy on the screen of the primary system.

According to the present example embodiment, primary intracorporeal ultrasonic imaging probe 100 has an ultrasonic imaging device (that consists of one or more ultrasonic imaging transducers in a known relative position and orientation configuration with respect to each other) that is configured to generate ultrasonic imaging energy (e.g. an acoustic imaging waveform) and ultrasonic communication energy (e.g. an acoustic communication waveform).

The ultrasonic communication energy is temporally triggered relative to the triggering of the ultrasonic imaging energy and the direction of propagation of the communication energy, which is referred to herein as the communication A-scan vector (or the communication A-scan, communication a-line, or communication vector), is spatially known relative to the direction of propagation of the imaging energy (also referred to as the imaging A-scan vector (or imaging A-scan, imaging a-line or imaging vector).

In the present example embodiment, primary intracorporeal ultrasonic imaging probe 100 is controlled by the control and processing system to generate both 2D and 3D ultrasonic imaging scans. A secondary intracorporeal device is placed into the patient's body and is arbitrarily positioned and oriented in relation to primary intracorporeal ultrasonic imaging probe 100.

The secondary intracorporeal device is equipped with one or more ultrasonic beacon transducers (also referred to as beacon transducers, or beacons) that are electrically connected to a secondary control and processing system (also referred to as a secondary system). This secondary control and processing system may be electrically isolated from the control and processing system or may be electrically connected to the control and processing system or may be physically integrated within the primary system). Each of the beacon transducers may be sensitive to its own range of ultrasonic operational frequencies for means of ultrasonic communication with primary intracorporeal ultrasonic imaging probe 100 (it is preferred that these beacon transducers have a broadband ultrasonic response and that the set of transducers covers as wide an ultrasonic acceptance angle as possible - this lends itself to polymer piezoelectric devices, but is not limited to just polymers). These beacons may operate with their own power supply, they may be wirelessly powered through inductive, ultrasonic or other energy harvesting means. The beacons may operate in an ultrasonic echo backscatter mode whereby demodulation techniques may be used to detect the presence of the ultrasonic echo backscatter waveform.

The secondary device is then moved such that its enclosure enters the scanning three-dimensional volume (also referred to as the Imaging Field of View) of primary intracorporeal ultrasonic imaging probe 100. If none of the beacons are within the Imaging Field of View of the primary intracorporeal ultrasonic imaging probe 100, then any 2-way ultrasonic imaging interaction between primary intracorporeal ultrasonic imaging probe 100 and secondary instrument is passive, and the secondary instrument may or may not be easily visible in the 2D and 3D images generated by the primary system. Visibility will depend on the relative orientation of the imaging A-scans originating from the device and the secondary instrument and the nature of the reflections and scattering of the transmitted imaging signal.

If at least one of the beacons is within either the imaging or the communication Field of View of the primary intracorporeal ultrasonic imaging probe 100, then an active ultrasonic communication link can be established by means of the operations performed by the secondary and primary control and processing systems. This link may be via: (I) an electrical path between the secondary system and the primary system, or (II) an ultrasonic path between the individual beacons that receive the transmitted ultrasonic waveforms from primary intracorporeal ultrasonic imaging probe 100 and retransmit a communication signal back towards primary intracorporeal ultrasonic imaging probe 100.

The system of primary intracorporeal ultrasonic imaging probe 100 receives ultrasonic imaging and communication information (by either said means of establishing an active communication link). The content of the imaging and communication information, via either the electrical path or the ultrasonic path, or both, may temporally coincide with the receipt of several imaging A-scan vectors (which are related both spatially and temporally to each other) and, for the case of the signals received along the ultrasonic path, may be of a different operational frequency and more generally of a different temporal waveform than what was initially transmitted by primary intracorporeal ultrasonic imaging probe 100.

For information received along the ultrasonic path, the control and processing system will filter and parse the received imaging and communication energy as required (spatially and temporally) and add a distinctive and visually appropriate signal (for example: a greyscale intensity profile or a coloured intensity profile) into the 2D and 3D imaging data presented to the user to help the user localize the secondary instrument.

The system of primary intracorporeal ultrasonic imaging probe 100, based on said filtered and parsed imaging and communication energy and information communicated via electrical paths between the primary and secondary intracorporeal devices may control the triggering and direction of subsequent A-scan vectors 115 (as shown in FIGs 6a-6d and Fig 8 to define a more narrow scanning three dimensional volume to track the position and orientation of the secondary instrument in space to allow the user to position the secondary instrument relative to the primary intracorporeal ultrasonic imaging probe 100 and more importantly, with respect to the operative procedure being performed, relative to tissue structures within the body. This action subsequently defines the relative position of the ultrasonic imaging device of primary intracorporeal ultrasonic imaging probe 100 with the beacons and any other component within the secondary instrument that is within the initial scanning field of view of the primary intracorporeal ultrasonic imaging probe 100.

This intracorporeal ultrasonic imaging system makes use of ultrasound signals and waveforms being generated by an ultrasound imaging transducer (acting as both transmitter and receiver) within a primary intracorporeal device or catheter. The ultrasound imaging transducer is in communication with a control and processing system that controls the timing of the transmitted signals, interprets received ultrasound signals (by means of amplifying, filtering, sampling and signal processing) and displays information on a display monitor for the user. The use of ASICs and switches for multiplexing in the event that more than one transducer stack is used within the primary device is also conceived. Although these components may be located within the probe body, they may also be provided within the primary processing and control system.

In transmit mode, the ultrasound imaging transducer generates and delivers ultrasonic energy along imaging vectors or A-scan directions, as well as ultrasound signals and waveforms along communication vectors that are of a fixed angle relative to the imaging A-scans and are of a known temporal relationship relative to the triggering of said imaging A-scans. This known temporal relationship is managed by the control and processing system 200 of primary intracorporeal ultrasonic imaging probe 100. This intracorporeal ultrasonic imaging system also makes use of ultrasonic beacon transducers that are integrated with secondary intracorporeal instruments, where the beacon transducers are ultrasonically responsive to a portion of the ultrasonic signals and waveforms associated with the imaging transducer of primary intracorporeal ultrasonic imaging probe 100, this includes imaging and ultrasonic communication signals transmitted from primary intracorporeal ultrasonic imaging probe 100. The beacon transducers are in communication with a secondary system associated with the secondary intracorporeal device. The secondary system is able to respond to the received ultrasonic communication signals in a manner that allows the control and processing system to utilize this response to identify the location and orientation of the secondary intracorporeal device within the 2D and 3D displayed image data.

The control and processing system controls the scanning and timing of ultrasonic transmission of the ultrasound imaging transducer in order to generate a set of A-scans. The set of A-scans defines one or more ultrasound Fields of View whereby the imaging transducer emits ultrasonic energy intended for more than one purpose. A first purpose is for imaging and a second purpose is for communication. The ultrasound band of frequencies used for imaging is referred to as the imaging band. The ultrasound band of frequencies used for communication is referred to as the communication band. These bands are independent of each other and may overlap in whole or in part or not at all.

By directing the orientation of successive A-scans, the control and processing system is able to define a 2D or 3D Field of View from within the body.

According to one example embodiment, the functional link between primary and secondary intracorporeal devices, controlled by the primary and secondary systems, consists of:
a) A timing clock used to trigger and control the time interval between transmitting imaging and ultrasonic communication signals during a system controlled sweep of imaging A-scans that make up the 2D area or 3D volume that is being ultrasonically interrogated.
b) The transmitting of a directed ultrasonic imaging energy. The electrical transmit signal used to generate the ultrasonic imaging energy having a characteristic temporal waveform that may be a single cycle, a number of cycles defining a tone burst, a modulated tone bursts, or a continuous wave. Each of these types of waveforms can be described by a transmit operational center frequency.
c) The transmitting of a directed ultrasonic communication signal. The electrical transmit signal used to generate the ultrasonic communication energy having a characteristic temporal waveform that may be a single cycle, a number of cycles defining a tone burst, or a continuous wave. Each of these types of waveforms can be described by a transmit operational center frequency.
d) The receiving of said directed ultrasonic waveforms by a beacon transducer on a secondary intracorporeal device that is positioned within either the imaging or the communication three-dimensional volume of the ultrasonic imaging device of the primary.
e) The issuing of a response to said received directed ultrasonic waveform by the system of the secondary instrument intended to be received by the system of primary intracorporeal ultrasonic imaging probe 100. The response may be issued by means of an electronic signal through one or more wires from the secondary instrument to the system of primary intracorporeal ultrasonic imaging probe 100, through one or more wires from the system of the secondary instrument to the system of primary intracorporeal ultrasonic imaging probe 100, or by means of an ultrasonic response initiated by the beacon transducer directed back to the transducer stack or stacks in primary intracorporeal ultrasonic imaging probe 100. Of these three configurations, the first two can be considered as a one-way active ultrasonic communication link and the third considered as a bidirectional active ultrasonic communication link.

For primary intracorporeal ultrasonic imaging probes where the directional imaging A-scans of the imaging transducer are able to cover a 3-dimensional volume and the position and direction of each A-scan is known relative to the longitudinal axis and transverse plane of the intracorporeal device (for example, as described in U.S. Patent No. 8,214,010) and whereby the 3D imaging volume includes forward viewing A-scans as well as more sideways viewing A-scans (where the component of the A-scan vector in line with the longitudinal axis is larger than the component of the vector in the transverse plane, and where the component of the A-scan vector in line with the longitudinal axis is smaller than the component of the vector in the transverse plane respectively), then the need for omnidirectional transducers within primary intracorporeal ultrasonic imaging probe 100, for means of triangulation to a positional reference frame that can be expressed in relation to the imaging transducer for means of defining the orientation of primary intracorporeal ultrasonic imaging probe 100 relative to the secondary instrument is not required.

The ultrasonic imaging device of primary intracorporeal ultrasonic imaging probe 100 may be made of a single transducer stack construction. More than one active piezoelectric layer may be electrically connected to a single electrical channel (e.g. a single coaxial cable), as described in PCT Patent Application No. PCT/CA2012/050057, titled "ULTRASONIC PROBE WITH ULTRASONIC TRANSDUCERS ADDRESSABLE ON COMMON ELECTRICAL CHANNEL". This may be achieved, for example, via pivots as shown in FIGS.. 5B, 5D, 5F, and 5H, or may be made of separate stack constructions.

In one example implementation, when two transducer stack constructions for multiple ultrasound transmitters are used in the primary intracorporeal ultrasonic imaging probe 100, a triaxial or twin axial cable may be used in lieu of a single coaxial cable to electrically connect said stack constructions with the added benefit of isolating the different RF signal paths while maintaining a small profile electrical wiring harness through the body of the intracorporeal device. If two transducer stack constructions are used, then it would be preferred that the stack constructions are mounted on a rigid assembly to maintain a fixed orientation between the directional transmitted ultrasonic waveforms generated by both transmitters, otherwise some sort of geodesic sensor or system could be used to correct for the variation in the relative directions of the transmitted ultrasonic waveforms.

The use of a single stack construction for the ultrasonic imaging device of primary intracorporeal ultrasonic imaging probe 100 is preferred in order to maintain a consistent directional relationship between the Imaging A-scans and the communication A-scans and has an added benefit that the intracorporeal ultrasonic imaging system can be realized without the need of multiplexing electrical signals within primary intracorporeal ultrasonic imaging probe 100 or the need for additional wires within the catheter construction. The imaging transmitter assembly may have more than one ultrasonically active layer within the structure. These ultrasonically active structures can be made of piezoelectric materials such as piezoelectric ceramics, piezoelectric ceramic and epoxy composite structures, piezoelectric single crystals, relaxor single crystal, relaxor ceramics, relaxor composite structures in a polymer matrix, or polymer piezoelectric layers such as PVDF or PVDF copolymers like P(VDF- TRFE), and can also be conceived of using CMUT and PMUT devices as well).

Due to the confined nature of the intracorporeal devices involved, the aperture size of the transmitting transducer of primary intracorporeal ultrasonic imaging probe 100 is limited. For an unfocussed transducer stack of a given aperture, the diffraction of the ultrasonic beam profile is less when the operational ultrasonic transmit frequency is higher, as shown in FIG.13B, FIG. 14, and FIG. 15. Therefore, when considering the selection of an operational ultrasonic communication frequency, the higher the operational frequency the better in terms of maintaining a lateral resolution of the directional ultrasonic communication energy being used to locate a secondary instrument (or conversely, the beacon of the secondary instrument will actively sense the communication energy over a smaller range of directional ultrasonic communication vectors). Given that ultrasonic penetration within the human body is stronger for lower frequencies, and in many cases it is desirable to image as deep into the body as possible, it is preferred (but not necessarily required) that the operational ultrasonic communication frequency be equal to or larger than the operational imaging frequency.

At higher frequencies, ultrasound energy is more readily attenuated in tissue. However, if the active the ultrasonic communication link is completed by means of an electrical wire from the beacon or from the secondary system back to the primary system, as shown in FIGS. 6A, 6B, and 6D, then the increased attenuation is only a one-way ultrasonic phenomenon as opposed to a two-way ultrasonic phenomenon and thus it is expected that the one-way active ultrasonic communication link can be established for distances that are greater than the deepest depth that can be imaged by the control and processing system if the imaging operational frequency were to be the same as the ultrasonic communication operational frequency.

Alternatively, the distances achievable for an active communication link can be increased by reducing the effects of ultrasound attenuation if the active ultrasonic communication link is completed by the beacon transducer sensing incoming ultrasound communication energy and the secondary system receives the incoming waveforms and transmits new ultrasonic communication energy that is intended to be received by the transducer of primary intracorporeal ultrasonic imaging probe 100. This is a bidirectional ultrasonic communication link wherein the intensity of the received electronic signal from the portion of the transducer stack construction intended to receive the ultrasonic communication energy will be stronger than if the communication was a two-way ultrasonic communication link resulting from the passive echo of the original ultrasonic communication energy bouncing off of the secondary instrument. In the case of the bidirectional ultrasonic communication path, the operating frequency and the temporal waveform of the return ultrasonic communication signal may be different than the initially transmitted ultrasonic communication energy.

It is further noted that although the example embodiments described above involve the use of a primary intracorporeal ultrasonic imaging probe that is configured to scan in three-dimensions while imaging, other example embodiments may involve two-dimensional scanning while imaging for the locating of a secondary device. For example, if it is known that a two-dimensional device lies within an imaging plane associated with a two-dimensional scanning imaging probe, then the methods disclosed herein may be employed to locate the secondary device in the two-dimensional plane.

The following examples are presented to enable those skilled in the art to understand and to practice embodiments of the present disclosure. They should not be considered as a limitation on the scope of the disclosure, but merely as being illustrative and representative thereof.

### EXAMPLES

For simplicity, the ultrasound beam profile for each imaging and communication A-scan is considered to be a fine line generated by a small aperture and the effects of diffraction are ignored.

### Example 1: Active Ultrasonic Communication Link Employing Mechanically Scanned Ultrasonic Imaging Transducer Using Non-Acoustic Communication

Primary intracorporeal ultrasonic imaging probe 100 emits a broadband imaging ultrasonic waveform with an operational frequency of 10 MHz. Primary intracorporeal ultrasonic imaging probe 100 houses a single element flat transducer stack with a tilt angle that depends on rotational speed and is rotating with a constant rotational speed such that the transducer tilt angle relative to the longitudinal axis of primary intracorporeal ultrasonic imaging probe 100 is fixed and the A-scan is fixed at 15 degrees relative to the longitudinal axis. The transducer is pulsing at a given pulse repetition frequency (PRF) and is therefore scanning a 2D image cone with a fixed cone angle of 15 degrees. This imaging energy is simultaneously used as an ultrasonic communication transmitted waveform. The waveform is sensed by a beacon transducer on a secondary instrument that is sensitive to 10 MHz ultrasonic energy. The beacon signal is received by the secondary instrument system and notification of the receipt of the ultrasonic energy is communicated to the control and processing system via a wire or cable using a defined communication protocol.

### Example 2: Active Ultrasonic Communication Link Employing Phased Array Using Non-Acoustic Communication

Primary intracorporeal ultrasonic imaging probe 100 emits a broadband imaging ultrasonic waveform with an operational frequency of 10 MHz. Primary intracorporeal ultrasonic imaging probe 100 houses a linear array transducer stack such that the elevation axis of the linear array is perpendicular to the longitudinal axis of the catheter and the 2D image plane of the array extends radially outwards relative to the transverse plane of the catheter. By phasing the transmit signals to the elements of the array, ultrasonic energy can be directed in A-scans that range from a preferentially forward looking direction to a preferentially backwards looking direction. By means of manual, electrical or robotic control, the catheter can be rotated in an alternating clockwise and anticlockwise fashion that defines a back and forth rocking motion. The largest three dimensional volume can be achieved when the rocking motion subtends 360 degrees>. If the rocking motion subtends less than 360 degrees then only a portion of the largest possible said 3D volume will be imaged. The timing between the phasing of the array elements and the rotational motion will determine the sequence of imaging A-scans used to sweep through the 3D volume. This imaging energy is simultaneously used as an ultrasonic communication transmitted waveform. The waveform is sensed by a beacon transducer on a secondary instrument that is sensitive to 10 MHz ultrasonic energy. The Beacon signal is received by the secondary instrument system and notification of the receipt of the ultrasonic energy is communicated to the control and processing system via a wire or cable using a defined communication protocol.

Other orientations of linear arrays are possible such as linear arrays that are in plane with the transverse plane of the catheter (the cross section of the catheter) or curvilinear arrays that are wrapped around a circumference that fits within the catheter.

### Example 3: Active In-Band Ultrasonic Communication Link Employing Mechanically Scanned Ultrasonic Imaging Transducer Using Acoustic Communication

Primary intracorporeal ultrasonic imaging probe 100 emits a broadband imaging ultrasonic waveform with an operational frequency of 10 MHz and the useful imaging bandwidth is between 7-13 MHz. Primary intracorporeal ultrasonic imaging probe 100 houses a single element flat transducer stack that is rotating in a manner consistent with what was described in Example 1 however instead of a fixed rotational speed, the motion is a defined ramp of rotational speed from an initial value of 20 rps to a final value of 70 rps, such that the transducer tilt angle relative to the longitudinal axis of primary intracorporeal ultrasonic imaging probe 100 varies from an initially sideways A-scan perspective to a final forward viewing A-scan perspective. The transducer is pulsing at a given PRF and is therefore directing imaging A-scans in a spiral manner that results in the scanning of a 3D image volume. It will be understood that the device may be configured to ramp the rotational speed, and thus ramp the rotational angle, up or down. This imaging energy is simultaneously used as an ultrasonic communication transmit waveform. The secondary instrument is positioned such that the waveform is sensed by more than one beacon transducer on a secondary instrument, where each beacon transducer is preferred to be broadband in nature (for example: a polymer PVDF device). Each beacon signal is received by the secondary instrument system and each beacon emits a narrowband response that is a combination of an operational frequency and a tone burst with a specific number of cycles (For example: consider 3 beacons that emit tone bursts of 8 cycles with an operational frequency of 8 MHz, 12 cycles with an operational frequency of 10 MHz, and 8 cycles with an operational frequency of 12 MHz respectively). The ultrasonic response of the primary transducer stack is also sensitive to receive at each of the beacon operational frequencies and thus primary intracorporeal ultrasonic imaging probe 100 system will be receiving 10 MHz imaging echoes from the backscattering of the tissue and will receive a different narrowband signal at a Time of Flight that is equivalent to the relative two-way distance between the primary transducer stack and each of the activated beacons attached to the secondary instrument plus a small delay equal to the latent response of the secondary system for each of the activated beacons. The detection of one or more of the operational frequencies associated with any of the beacons will indicate that a secondary instrument is present within the imaging field of view of the primary intracorporeal ultrasonic imaging probe 100. The combination of all of the received communication signals will permit a better determination of the orientation of the secondary instrument relative to primary intracorporeal ultrasonic imaging probe 100. This detection will occur within the individual temporal windows of each of the directional imaging A-scans.

### Example 4: Active Dual-Band Ultrasonic Communication Link Employing Mechanically Scanned Ultrasonic Imaging Transducer Using Acoustic Communication and Diffraction Grating Enhancement of Communication Scanning Vectors

Primary intracorporeal ultrasonic imaging probe 100 emits a broadband imaging energy with an operational frequency of 10 MHz. Primary intracorporeal ultrasonic imaging probe 100 is rotating with a constant rotational speed in a manner consistent with what was described in Example 1, such that the transducer tilt angle relative to the longitudinal axis of primary intracorporeal ultrasonic imaging probe 100 is fixed and the A-scan is fixed at 40 degrees relative to the longitudinal axis. The transducer stack is pulsing at a given PRF and is therefore scanning a 2D image cone with a cone angle of 40 degrees relative to the longitudinal axis of the primary catheter. The transducer stack of primary intracorporeal ultrasonic imaging probe 100 is also simultaneously emitting a more narrowband ultrasonic communication energy (a tone burst of say 8 cycles) with an operational frequency of 30 MHz with the same PRF and with 0s time lag relative to the imaging energy such that the imaging and communication waveforms are superimposed. The ultrasonic communication energy is transmitted in at least three principle directions by means of an ultrasonically active diffraction grating embedded within the primary transducer stack. One of the three ultrasonic communication direction vectors is parallel and coincident with the imaging A-scan of the 2D image cone and the other two are in directions that both lead and lag the imaging A-scan by an angle of +24.6 degrees and -24.6 degrees respectively and also lie on the surface of the 2D image cone. The secondary instrument is a straight instrument and is positioned in a manner relative to the primary catheter such that the instrument is tangential to the 2D image cone and only the 'leading' ultrasonic communication pulse at +24.6 degrees is incident on the secondary intracorporeal device and a beacon happens to be positioned at the location of incidence. The beacon signal is received by the system of the secondary instrument and a 30 MHz operational frequency tone burst of 12 cycles is retransmitted by the same beacon. The primary transducer stack, which is sensitive to receive at both 10 and 30 MHz operational frequencies, will be receiving imaging echoes at 10 MHz and will detect a 30 MHz ultrasonic waveform that does not coincide with a similar imaging echo (unless by chance there is tissue at an equal time of flight distance away along the imaging A-scan vector with a large enough change of acoustic impedance to emit a noticeable echo different than the background scattering level). After a time interval of a plurality imaging A-scan vectors (the temporal relation is dependent on the PRF and the rotational speed of the transducer), the transducer will have rotated such that the direction of the imaging A-scan is now approximately 24.6 degrees from the initial imaging A-scan vector in this example. At this moment in time, the primary transducer will receive an imaging echo and an ultrasonic communication energy (that lags by the time delay in the secondary system to process the received 30 MHz waveform and transmit the response waveform). After a second interval of a plurality A-scan vectors, the transducer has rotated such that the imaging vector is now 24.6 degrees 'ahead' of the secondary instrument. The primary transducer, which is sensitive to receive at both 10 and 30 MHz will be receiving imaging echoes at 10 MHz and will detect a 30 MHz ultrasonic signal that does not coincide with a similar imaging echo. This sequence of events, as sensed within a set of received RF lines of the intended 2D image cone helps to confirm the existence of a beacon device within the field of view of the primary imaging device. This example can be extended to consider a 3D imaging volume by ramping the rotational speed of primary intracorporeal ultrasonic imaging probe 100 and thus sweeping through a range of cone angles. For cone angles larger than 40 degrees, each ultrasonic A-scan will be incident on the secondary instrument twice. The second location of incidence may or may not coincide with a beacon. For cone angles smaller than 40 degrees, none of the ultrasonic A-scans will be incident on the secondary instrument. By correlating the timing of events with the spatial knowledge of each imaging A-scan of each event (an event being the identification of either a passive imaging echo from the secondary intracorporeal device or the identification of an active ultrasonic communication signal by either an electrical wire or an acoustic path all of which originating from the secondary intracorporeal device), the position and orientation of the secondary intracorporeal device can be identified.

### Example 5: Active Dual-Band Ultrasonic Communication Link Employing Mechanically Scanned Ultrasonic Imaging Transducer Using Acoustic Communication and Echo-Backscattering Mode

The positional configuration of primary intracorporeal ultrasonic imaging probe 100 and secondary instrument of the preceding example is revisited with primary intracorporeal ultrasonic imaging probe 100 emitting a broadband imaging energy with an operational frequency of 10 MHz. Primary intracorporeal ultrasonic imaging probe 100 is also simultaneously emitting a more narrowband ultrasonic communication energy of operating frequency of 30 MHz with 20 cycles with the same PRF and with a time lag of 40 usec, which, if propagating in water, translates into about a 6 cm 1-way distance lag between the imaging energy and the ultrasonic communication energy. The beacon transducer and secondary system is configured to operate in an ultrasonic Echo Backscatter mode. A beacon transducer used in this manner is being operated in a semi-passive mode. The ultrasound communication is established by a standard backscatter modulation similar to a radio-frequency identification device. Backscatter modulation can result from changing the impedance of the beacon transducer by means of a switch that either shorts the electrical impedance of the beacon transducer or leaves the beacon transducer at its natural electrical impedance. For a more detailed explanation, one can refer to the published paper by Mari et al. "Doppler detection of deeply implanted impedance-switching devices Using Ultrasound Doppler" published in IEEE Transactions on Ultrasonics, Ferroelectrics, and Frequency Control, vol. 60, no. 6, Pages 1074-1083 June 2013.

In this example, switching frequency of 13 MHz between open and shorted states of the beacon transducer is used. This will result in an amplitude modulated return signal that contains 8.67 periods of modulation. This modulated signal, at 30 MHz would be detected when each of the diffraction lobes of the diffraction grating are directed towards the beacon device. The echo backscatter would not be detected by the imaging energy when the imaging energy is directed towards the beacon since the pulse duration of the imaging energy is shorter than the period of the switching mechanism of the beacon echo backscattering circuitry.

### Example 6: Transducer Beam Profile

The actual choice of imaging and ultrasonic communication operational frequencies will depend on different parameters. For example, when considering the intended use of the primary and secondary intracorporeal devices, there may be preferential relative distances and orientations that have a higher likelihood of occurring and this may impact the choice of using a flat or curved transducer. In addition, the actual beam profile of the transducer of primary intracorporeal ultrasonic imaging probe 100 will depend on the medium that is being imaged (attenuation and scattering will alter the beam profile).

To demonstrate these design choices, a simple beam profile equation can be used to demonstrate the principle involved for a flat transducer, as illustrated in FIG. 13A. The solid lines show an idealized -6dB beam profile for a circular transducer with a radius (a) operating at a frequency (F). The outline of the profile is defined by the solid lines in the figure. In the near field of the transducer the beam profile is equal to the aperture of the transducer and in the far field the diffraction of the beam shows how the beam profile spreads as a function of distance away from the transducer. The diffraction angle alpha of the beam profile is a function of the radius of the aperture and the operational frequency of the transducer. The equation is shown in FIG 13A. If the aperture were rectangular, then the equation would apply in both principle axes of the transducer and the diffraction of the beam would be different in each principle axis. This can be extended to transducers of a larger number of sides.

For example, an ICE imaging system that displays imaging data for a depth of up to 6cm and utilizes a primary intracorporeal ultrasonic imaging probe with a flat transducer that has an aperture of 2mm in diameter and an imaging operational frequency of 10 MHz would have a -6dB beam profile of about 9 mm in diameter at a depth of 6cm. This is in contrast to an ultrasonic communication signal in the range of 20-30 MHz, with an equivalent 2mm aperture which would have a beam profile of about 5-3mm in diameter respectively. The shape of the aperture of the flat transducer can be circular, elliptical, square, rectangular, or some other arbitrary shape that can be patterned by material processes that are either additive or subtractive in nature, however for simplicity, a circular aperture is considered here.

FIGS. 13B shows a plot of the 6 dB beam profile of 10 MHz transducer with 2mm diameter aperture. FIGS. 14 shows a plot of the 6 dB beam profile of 30 MHz transducer with 2mm diameter aperture. The beam profile is better maintained at the higher frequency. FIG. 15 plots the 6 dB beam profile of 20 MHz transducer with 2mm diameter aperture. All axes are in mm. Beam profile at 6cm in depth is 4.6mm in diameter.

### Example 7: Single Stack Ultrasonic Transducers having Separate Imaging Band and Communication Band

In the following section, three example embodiments of ultrasound imaging transducer stacks provided as exemplary concepts for the ultrasonic imaging device of the primary intracorporeal ultrasonic probe that can be used to realize the transmission and reception of the ultrasonic signals used for the ultrasonic communication band of the embodiments of this invention. Specific examples of each concept will follow where the configuration of the electrical leads of the transducer stack are presented in a manner that permits all exemplary transducer stacks to be integrated into a 10French catheter of the type described in U.S. Patent No. 8,214,010. These examples are not limited to 10 French and can be used in smaller and larger sized catheters typically used to house imaging transducers in the 4-100 MHz range and in hand held ultrasound probes that can range from 0.5 -100 MHz. All of the specific examples apply the usual rules in transducer stack design known by individuals skilled in the art, where matching layers are ¼ of an operational wavelength in thickness based on the speed of sound of the material and the piezoelectric layer thickness ranges between ¼ and ½ of an operational wavelength and is determined based on the mass loading of the piezoelectric layer due to the acoustic impedance of the adjacent layers. The thickness of the backing will be limited due to space constraints within primary intracorporeal ultrasonic imaging probe 100.

The following section describes three example single-stack ultrasound transducer configurations:
(i) A baseline imaging stack using a single piezoelectric layer with matching and backing layers used to create a reasonable bandwidth for imaging purposes. In this case, the imaging transmit pulse may also be used as the communication signal, or a portion of the imaging bandwidth of the piezoelectric layer may be used to generate a characteristic communication signal. Other resonant modes or harmonics of the piezoelectric layer may also be used.
(ii) A modified imaging stack where the single piezoelectric layer is replaced with an active piezoelectric layer and an ultrasonically heavy support, which may be an unpoled piezoelectric layer or a passive layer of comparable acoustic impedance (such as a metal, or any other ceramic or single crystal (lithium niobate, unpoled PMN-PT, etc...) to the active piezoelectric layer. A stack of this nature can preserve the original imaging band of the transducer while creating an additional resonant frequency band that can be utilized for communication. The electrode aperture of the two frequency bands are identical.
(iii) A dual piezoelectrically active imaging stack where one of the ultrasonic coupling layers (i.e. matching layers) is a thin piezoelectric layer. Due to size constraints within the primary intracorporeal device, it is preferred that the additional piezoelectric layer share common electrodes with the imaging piezoelectric layer such that both layers are electrically connected in parallel with each other and can be electrically driven by the means of a single electrical connection to the stack. It is noted that the ultrasonic coupling layers may be of a quarter wavelength design or of a mass-spring type design. A stack of this nature can preserve the original imaging band of the transducer while creating an additional resonant frequency band, with an independent aperture that can be utilized for communication.

FIG. 16 shows a baseline imaging transducer stack 300 that is suitable for 2D and 3D ultrasound imaging in a primary catheter device that can be fabricated to have an imaging operational frequency in the range of 0.5-100 MHz or more commonly in the range of 4-60 MHz. The figure is an isometric drawing showing a perspective of the stack and three faces of the stack. The arrows 305 indicate the intended direction of the transmitted imaging energy. The lower left side view is drawn such that the top face of the transducer is pointing downwards meaning that the imaging A-scan vector is intended to propagate downwards. The transducer stack consists of a single piezoelectric layer 310 with thin metal electrodes (example: chromium/gold or titanium/tungsten) on the top and bottom face (not shown in the figure). On top of the transducer are two matching layers where each matching layer is ¼ wavelength thick relative to a desired operational frequency. The first matching layer 315 is made of electrically conductive epoxy and the second layer 320 is made of non-conductive epoxy. On the bottom face of the transducer a conductive epoxy layer is used as a backing layer 325. The backing layer 325 is of limited thickness such that the total thickness of the transducer fits inside the catheter. On the left side of the piezoelectric there exists a vertical epoxy dielectric layer 330 with a further vertical electrically conductive epoxy layer 335 that defines the outer face of the transducer. The dielectric layer 330 provides electrical isolation between the bottom and top faces of the piezoelectric layer 310 and allows for electrical connection to the transducer stack from the left and right side of the stack as seen in the lower left view.

FIG. 17 shows an isometric drawing of the modified imaging transducer stack that is suitable for 2D and 3D ultrasound imaging in a primary catheter device that can be fabricated to have an imaging operational frequency in the range of 0.5-100MHz or more commonly 4-60MHz. The arrows 405 indicate the intended direction of the transmitted imaging energy. The stack is drawn such that the top face of the transducer is pointing downwards meaning that the imaging A-scan vector is intended to propagate downwards. The transducer stack consists of a single active piezoelectric layer 410 with thin metal electrodes (example: chromium/gold or titanium/tungsten) on the top and bottom face (not shown in the figure). On the bottom face of the active piezoelectric layer is a second inactive layer 412 of the same PZT material. The inactive layer is thinner than the active layer. Due to the mass loading from this inactive layer, the active piezoelectric layer needs to be thinner than in the baseline imaging transducer stack example to achieve similar operational frequencies.

On top of the active piezoelectric layer 410 are two matching layers where each matching layer is ¼ wavelength thick relative to the desired operational frequency. The first matching layer 415 can be made of electrically conductive epoxy and the second layer 420 is made of non-conductive epoxy. The ultrasonic impedance of the first matching layer 415 was increased to about 8 Mrayls from the value of about 5.9 Mrayl used in the baseline imaging transducer stack and was necessary to help maintain a similar ultrasonic excitation response as said baseline imaging transducer stack. The increased acoustic impedance can be achieved, for example by adding some tungsten powder to the silver epoxy (or alternatively, the top metal electrode can be made thicker (0.6-1.5 um in thickness) and a tungsten loaded or alumina loaded non-conductive epoxy mixture can be used). On the bottom face of the transducer, a conductive epoxy layer is used as a backing layer 425. The backing layer 425 is of finite thickness such that the total thickness of the transducer fits inside the catheter. On the left side of the piezoelectric there exists a vertical dielectric layer 430 with a further vertical electrically conductive epoxy layer 435 that defines the outer face of the transducer. The dielectric layer 430 provides electrical isolation between the bottom and top faces of the piezoelectric layer 410 and allows for electrical connection to the transducer stack from the left and right side of the stack.

FIGS. 18A and 18B compare the simulated electrical impedance response of the baseline 300 and modified 400 imaging stacks with an imaging operational frequency of 9.4 MHz for a transducer of rectangular aperture equal to 2.05mm x 1.85mm. This simulation was performed with consistent ultrasonic loads and consistent and ideal 50 Ohm electrical loads in order to isolate the effects of the different ultrasonic stacks. The primary Y axis of the graph, on the left, shows the magnitude of the electrical impedance of the stack and the secondary Y axis on the right shows the phase response of the stack and both are plotted as a function of frequency in MHz.

The baseline imaging stack shows two resonant peaks at frequencies below 50 MHz with a fundamental resonance in the 9-10 MHz range and a 3^{rd} harmonic in the 34-38 MHz range. The modified imaging transducer 400 shows three peaks at frequencies below 50 MHz with a pair of resonances below 25 MHz (in the 8-12 MHz and 20-25 MHz range) related to the interaction of the two PZT layers (410 and 412) and the fundamental resonance of the active PZT. The electrical impedance at these resonant frequencies are each better matched to 50 Ohms than the fundamental in the baseline imaging stack and, in this case, will allow for a better electrical transfer of energy from the transmitter to the electrical load of the transducer. The ratio of the thicknesses of the poled 410 and 'unpoled' PZT 412 can be in the range of 1.5-4 and will result in a determined pair of resonant frequencies. The actual ratio of the simulated response of the stack is 2.8. As the ratio changes so do the determined pair of resonant frequencies. The higher resonance is intended to be used for ultrasonic communication and in the event of a bidirectional ultrasonic communication link, the operational frequency of the transmitted beacon waveform should be equal. The use of memory on primary intracorporeal ultrasonic imaging probe 100 allows for the actual pair of frequencies to be stored and the use of a broadband beacon transducer can be used to accommodate the variation in the range of the ratio of poled 410 to unpoled 412 PZT. The third frequency is in the range of 42-46 MHz.

The same simulation is extended, in FIGS. 19A and 19B, to compare the two-way excitation response of the baseline imaging transducer stack 300 and the modified imaging transducer stack 400. The excitation transmit signal was a broadband single cycle sinusoidal bipolar pulse with a center frequency of 10 MHz. The time response waveform is plotted as the primary Y-axis of the graph as a function of time as a linear ratio of Voltage out (in Rx) / Voltage in (in Tx). The spectrum of the time response is plotted as the secondary Y axis as a function of frequency as a decibel ration as a ratio of Voltage out (in Rx) / Voltage in (in Tx). The amplitude response ends up being a little stronger for the modified imaging transducer stack 400 than for the baseline imaging transducer stack 300.

Some of the ultrasonic parameters of the 1-way excitation response for both stacks are summarized in FIG. 20. The table shows that the primary imaging resonance at 9.4 MHz is preserved for both stacks with comparable -6dB and -20 dB bandwidth and pulse responses respectively.

FIG. 21 shows an example of a dual piezoelectrically active imaging stack 500 that is suitable for 2D and 3D ultrasound imaging in a primary catheter device with an imaging operational frequency that can be fabricated to have an imaging operational frequency in the range of 0.5-100 MHz and preferably 4-60 MHz. The stack 500 is drawn such that the top face of the transducer is pointing downwards meaning that the imaging A-scan vector is intended to propagate downwards. The arrows 505 indicate the direction of the imaging energy. The transducer stack 500 consists of a PZT piezoelectric layer 510 with thin metal electrodes (Example: chromium/gold or titanium/tungsten or other suitable mixed metal formulations) on the top and bottom face. On top of the PZT layer 510 are three layers that form the matching layer structure. The first is the initial ¼ wave matching layer 515 and is electrically conductive. The second layer is a 9um PVDF piezoelectrically active polymer layer 518 with thin metal electrodes (Example: chromium/gold) on its bottom and top face. The third layer is a low ultrasonic impedance material 520. In effect, the combined thickness of the PVDF 518 and the third layer 520 can be thought of as a composite layer that acts in a manner that is consistent to the second matching layer 520 of the baseline imaging transducer. The electrodes of the PVDF layer should preferably be thin (less than 1 um) to avoid reflection effects. For a 10 MHz imaging transducer, using 301 epoxy from Epoxy Technology as the third layer 520 in the matching layer structure, a 9um PVDF layer 518 is about 13.5% of the ¼ wavelength of a pure single phase 301 epoxy layer. The thickness of the actual 301 is then made thinner to compensate for the presence of the PVDF. On the bottom face of the transducer a conductive epoxy layer is used as a backing layer 525. The backing layer is of finite thickness such that the total thickness of the transducer fits inside the catheter. On the left side of the piezoelectric there exists a vertical dielectric layer 530 and to the outside of that is a vertical electrically conductive layer 535 that defines the outside perimeter of the transducer stack 500. The dielectric layer 530 provides electrical isolation between the bottom and top faces of the piezoelectric layer 510 and allows for electrical connection to the transducer stack 500 from the left and right side of the stack.

The metal electrodes of the polymer layer 518 are electrically connected to the electrodes of the imaging transducer such that the piezoelectric layers are electrically in parallel with each other. This requires an additional pair of vertical layers 540 and 545, both an inner dielectric layer and an outer conductive layer, similar to the existing pair described in the baseline imaging stack. The additional pair of vertical layers can be at an angle relative to the initial pair of vertical layers. For simplicity, the additional pair of vertical layers are shown to be perpendicular to the original pair. The additional vertical dielectric layer 540 does not extend all the way through the backing layer to allow for the top electrode of the PVDF to be in electrical communication with the bottom electrode of the PZT. In this configuration, the electric field applied across the PZT will be opposite in direction to the electric field applied across the polymer 518.

A metal foil bridge 560, that is soldered or glued with conductive epoxy to the top electrode of the polymer, electrically connects the top electrode of the polymer piezoelectric to the second outermost electrically conductive layer. This can be seen in the expanded detail to the right of the figure. This bridge could equally be formed by vacuum deposition techniques such as evaporation or sputtering or with conductive epoxy (an equivalent example 660 is shown in the detail A of FIG. 24).

FIGS. 22A and 22B compare the simulated two-way excitation response of the baseline imaging transducer stack 300 and the dual piezoelectrically active transducer stack 500 for a stack intended to have an operational frequency of close to 10 MHz with a rectangular stack aperture of 2.05mm x 1.85mm. This simulation was performed with consistent ultrasonic loads and consistent and ideal 50 Ohm electrical loads in order to isolate the effects of the different ultrasonic stacks. In this case the acoustic impedances of the matching layers were not altered. The excitation transmit signal was a broadband sinusoidal single cycle bipolar pulse with a center frequency of 10 MHz. The time response waveform is plotted as the Primary Y-axis of the graph as a function of time as a linear ratio of Voltage out (in Rx) / Voltage in (in Tx). The spectrum of the time response is plotted as the secondary Y axis as a function of frequency as a decibel ration as a ratio of Voltage out (in Rx) / Voltage in (in Tx). The amplitude response is a little stronger for the baseline imaging transducer stack 300 than the dual piezoelectrically active transducer stack.

Some of the ultrasonic parameters of the 2-way excitation response for both stacks are summarized in FIG 23. The effects of inserting the PVDF layer 518 into the matching layer structure of the stack are minimal and are considered to be comparable.

The dual piezoelectrically active transducer stack 500 provides the additional option of patterning the top electrode of the PVDF layer to define a diffraction grating structure. The use a grating structure allows for more than one direction of ultrasonic communication and for directions that are fixed relative to the ultrasonic imaging A-scans that are created by the lower frequency PZT piezoelectric layer. Linear and 2D diffraction gratings are possible. For simplicity, the case of 2D diffraction shall be discussed towards the end of the document.

An example of the top electrode pattern 650 for the PVDF is shown within FIG. 24 (with the uppermost layer of the stack removed). This pattern consists of a number of rectangular electrodes 655 that have a center to center spacing (d) and a gap (w) between nearest neighbours. All of the metal electrodes are electrically shorted together by means of a metal bridge 660, so the PVDF is acting as a single element device.

The device will behave in a manner similar to a linear array where all elements are electrically driven with 0 degrees of phase. The plane of the patterned metal electrode has two principle axes. The axis parallel to the length of the metal strips is referred to as the elevation axis, and the axis perpendicular to the elevation axis is referred to as the azimuth axis.

There is no beam forming capability as this is a single element device. Given the center to center spacing between nearest neighbouring metal strips, there are angles (alpha) where constructive interference occurs. This can be seen in FIG. 25. These angles of constructive interference coincide with when the path length difference between propagating ultrasonic energy from neighbouring elements is equal to an integer multiple of the propagating wavelength within the propagating medium. For the case of a catheter in the heart, this medium is modelled as blood. Although there may be several angles of constructive interference, in practice, the predominant lobes are the ones that are closest to being normal to the plane of the grating structure due to the directivity pattern of each effective piezoelectric element. If the grating structure is simply a patterned electrode, then the structure is similar to a kerfless array and the amount of ultrasonic energy propagating at large oblique angles relative to the normal will be greatly reduced relative to the amount of energy propagating in a direction normal to the plane of the structure. A portion of the piezoelectric material, in between the metal electrodes, can be removed to increase the off-axis energy emitted from the grating structure and reduce the directivity of the overall grating structure. These kerfs will be filled by the epoxy material that makes up the balance of the second matching layer, or if the top layer is a preform layer, by the adhesive used to bond the top layer to the stack.

Although not shown, the dual piezoelectrically active transducer stack can also be implemented using a heavier piezoelectric material that is incorporated into the first matching layer in a manner consistent with the principles described above. For such a transducer stack, the first matching layer would be a combined layer of a heavy conductive metal layer (for example silver epoxy or plated gold on top of the thin electrode connected to the top surface of the imaging piezoelectric layer) and a second heavy piezoelectric (made from PZT, porous PZT, PZT/epoxy composite materials of a controlled acoustic impedance to match the electrically conductive layer below, other piezoelectric ceramics and composites using lithium niobate, relaxor materials, etc...). The metal bridge would still be located under the second matching layer. The top matching layer could now be a single phase material of a specific acoustic impedance suitable for coupling the imaging signals and waveforms out of the stack. It is also conceived that the matching layers that are not build up with an active piezoelectric layer can also be made with graduated acoustic impedance that varies along its thickness axis and such a graduated matching layer design can be applied to any of the presented stack concepts and examples.

To explore the relationship of the diffraction grating electrode pattern with angles of constructive interference, consider a diffraction grating transducer designed to operate at 30 MHz. If the spacing (d) between metalized strips is 120um and the gap between the strips is 60um then the first lobe of constructive interference is 24.6 degrees for a propagating medium of blood. The gap between the electrodes is a little more than a wavelength which is 52um at 30 MHz in blood.

FIG. 26 shows a logarithmic beam profile for a 2D linear ultrasonic wave propagation simulation. The beam plot shows the maximum temporal pressure along the perpendicular plane that bisects the elements of the grating structure. The diffraction grating structure consists of 14 electrodes and the transmitted tone burst consists of 14 cycles. The lengths of the electrode strips are into the page. In order to have an appreciable amount of energy in the off axis lobes, it is necessary to ensure that the transmit pulse has enough cycles such that there is temporal overlap between the ultrasonic waveforms emitted from the metal strips at the extreme ends of the structure. If this condition is not met, then the peak intensity in the off axis lobes will be reduced and the lobe will appear to be more diffuse as there is less constructive interference taking place. It is noted that a transmit tone burst of more or less cycles can be used as part of the confirmation of the ultrasonic communication link if that information is known by the control and processing system and if the secondary instrument or secondary system relays this information back to the control and processing system by any of the methods disclosed.

The geometric design and transmit conditions of the diffraction grating provide an angular dependent behaviour of the diffraction grating beam that can be taken advantage of in 3D imaging to actively communicate with the set of beacon sensors of a secondary instrument as a function of imaging A-scan orientation and time.

The orientation of the diffraction grating can be defined relative to the scanning direction of the imaging transducer. If the elevation axis is perpendicular to the scanning direction of the imaging transducer, then the grating lobes can provide an early indication of the presence of the secondary instrument. This configuration was described in example 4. If the elevation axis is parallel to the scanning direction of the imaging transducer, then the grating lobes (other than the normal lobe) are off axis relative to the scanning plane and provide additional opportunities to identify beacon transducers attached to secondary instruments.

The orientation of the diffraction gratings 650 and 750 shown in FIGS. 24 and 27A, if inserted into the a catheter of the type described in U.S. Patent No. 8,214,010, is such that the elevation axis of the diffraction grating transducer is parallel to the tilt axis of the transducer. The tilt axis is a relative reference frame with respect to the longitudinal axis of the catheter.

In contrast, the orientation of the diffraction grating 850 shown in FIG. 27B, if inserted into a catheter of the type described in U.S. Patent No. 8,214,010, is such that the elevation axis of the diffraction grating transducer is 800 perpendicular to the tilt axis of the transducer. The tilt axis is a relative reference frame with respect to the longitudinal axis of the catheter. FIG. 27A also shows that if the corners 770 of the transducer aperture are cut off to make an eight sided shape that the second vertical dielectric layer 740 does not need to extend to the back face of the backing 725 to maintain electrical isolation between the two piezoelectric layers and the two vertical electrically conductive layers that make up the electrical leads of the transducer stack.

With respect to the catheter of the type described in U.S. Patent No. 8,214,010, the elevation axis parallel to the scanning direction is of special interest because the grating lobes will define additional effective tilt angles for ultrasonic communication that, in general, follow cones of small and larger angles than the imaging cone angle. This means that when the tilt angle of the imaging transducer is swept to create a spiral 3D scan, beacons beyond the range of the imaged 3D volume can still be detected by means of ultrasonic communication. This is a preferred orientation for the grating lobes. This configuration is shown in FIG. 28, in which dual band diffractive imaging transducer stack 800 is shown supported within imaging assembly 890, where diffractive orders 880 are shown emitted by the diffraction grating structure. A final example of ultrasonic communication link is provided below.

### Example 8: Mechanically Scanned Ultrasonic Imaging Transducer Using Acoustic Communication and Diffraction Grating Enhancement of Communication Scanning Vectors

Primary intracorporeal ultrasonic imaging probe 100 emits a broadband imaging energy with an operational frequency of 10 MHz. Primary intracorporeal ultrasonic imaging probe 100 is rotating with a constant rotational speed such that the transducer tilt angle relative to the longitudinal axis of primary intracorporeal ultrasonic imaging probe 100 is fixed and the A-scan is fixed at 40 degrees relative to the longitudinal axis. The transducer stack is pulsing at a given PRF and is therefore scanning a 2D image cone of a fixed angle of 40 degrees relative to the longitudinal axis of the primary catheter. The transducer stack of primary intracorporeal ultrasonic imaging probe 100 is also simultaneously emitting a more narrowband ultrasonic communication energy (a tone burst of 14 cycles) with an operational frequency of 30 MHz with the same PRF and with 0s time lag relative to the imaging energy. The ultrasonic communication energy is transmitted in at least three principle directions by means of a piezoelectrically active diffraction grating embedded within the primary transducer stack. (For the sake of simplicity, the rest of this example will only consider 3 principle directions: the -1, 0, +1 directions). One of the three ultrasonic communication direction vectors is parallel and coincident with each imaging A scan of the 2D image cone and the other two are in directions that are equivalent to an effective transducer tilt angle of 40+24.6= 64.6 degrees and 40-24.6=16.4 degrees respectively. This means that the ultrasonic communication signals simultaneously scan three 2D cones at once.

The secondary instrument is positioned in a manner relative to the primary catheter such that a beacon is in the beam profile of the 64.6 degree cone and portions of the secondary instrument are also in the ultrasonic path of the 40 degree and 16.4 degree cones. The beacon signal is received by the system of the secondary instrument and a 30 MHz operational frequency tone burst of 20 cycles is transmitted. The primary transducer stack, which is sensitive to receive at both 10 and 30 MHz operational frequencies, will be receiving imaging echoes at 10 MHz and will detect an active bidirectional ultrasonic communication signal at 30 MHz. Depending on the actual relative orientation of the secondary instrument, the primary stack transducer may also receive passive echoes at 10 MHz and 30 MHz during subsequent imaging A-scans that coincide with the secondary instrument being in the ultrasonic path of the transmitted waveforms from primary intracorporeal ultrasonic imaging probe 100.

This example can be extended to consider a 3D imaging volume by ramping the rotational speed of primary intracorporeal ultrasonic imaging probe 100. The system, controlling primary intracorporeal ultrasonic imaging probe 100, can keep track of the A-scans where active ultrasonic communication signals are detected and can cross reference these events to determine which one of the grating lobes communicated with each beacon that was present in the 3D imaging scan and can more effectively determine the location and orientation of the secondary instrument than by relying on the imaging A-scans alone. The control and processing system can then optionally redirect the imaging transducer to scan through a subset of the 3D imaging volume to sample the active communication events more frequently and therefore better determine the relative position of the secondary intracorporeal device if the device is stationary, or track the movement of the secondary instrument if it is being manipulated by the user. Furthermore, in one example embodiment, the subset of the 3D imaging volume may be a 2D plane that includes the position of the secondary intracorporeal device.

It is noted that with the diffraction grating oriented in this manner, there are a few special cases where the diffraction grating maps out less than 3 cones. If the imaging transducer is tilted such that the imaging A-scan angle is equal to 24.6 degrees from the longitudinal axis of the catheter then one diffraction lobe will be pointed straight forward, parallel to the longitudinal axis of the primary catheter, and the other two lobes will map out cones of angles 24.6 an 49.2 degrees. If the imaging A-scan angle is 12.3 degrees then two of the diffraction lobes will map out a cone of angle 12.3 degrees and the third will map out a cone of angle 36.9 degrees. If the imaging A-scan angle is 0 degrees, then the central lobe will be pointing straight forward and the other two lobes will map out a cone of half angle 24.6 degrees.

The diffracting grating concept can be broadened to consider a 2D diffraction grating were an addition set of metal bars are patterned in conjunction with (or on top of) the existing metal bars but at an angle relative to the existing metal bars (for example: at 90 degrees, but the angle may be less than 90. It should be noted that the angle of 0 degrees is not particularly useful as this is the same direction as the existing metal bars. A 2D grating structure would eliminate the need of differentiating between the said two principle orientations already described above through Examples 4, 5, 7, and 8 as well as shown in Fig. 28 since the metal strips of a 2D diffraction grating would extend in both elevation and azimuth and the common shorting bar features in FIGS. 27A and 27B would not be required. The diffraction grating lobes can be projected along both principle axes at the same time with the increased functionality of tracking more communication A-scans. The angles of the - 1,0,1 lobes of each primary axis do not necessarily need to be at the same angles. This means that the spacing (d) and gaps (w) between adjacent metal strips for each principle axis of the 2D structure do not need to be the same. If a 2D diffraction grating is implemented, then the primary control and processing system would be simultaneously searching for the types of communication events described in Examples 4,5 and 8 combined.

### Example 9: System and Beacon Characteristics

By means of an EEPROM and permanent memory, it is possible to configure the parameters of the ultrasonic communication in advance of the intended use. A priori knowledge of the nature of the ultrasonic communication aids in the handshaking between the control systems associated with primary intracorporeal ultrasonic imaging probe 100 and the secondary instrument. The ultrasonic communication can be defined by parameters that include, but are not limited to, the choice of primary intracorporeal ultrasonic imaging probe 100's 1-way pulse excitation response (broadband or narrowband signals, amplitude or frequency modulated signals, the use of a DC offset in the excitation signal, etc.), the Pulse Repetition Frequency (PRF) of the excitation, the nominal strength of the excitation of the transducer stack, or stacks, within primary intracorporeal ultrasonic imaging probe 100. The same is true for the intrinsic response of the beacon, or plurality of beacon transducers, in the secondary instrument and this includes the received and transmitted directional sensitivity of the beacon circuitry, the choice of operational frequencies of each beacon, the 1-way pulse excitation response, the location of each beacon on a secondary instrument and the spatial relationship between the beacons can all be known a priori. These parameters (and others) can be stored in memory and with the parameters of the communication band defined and accessible by the primary control system, they can be used to establish an active communication link between the primary catheter and the secondary instruments. This handshaking can help prevent misuse (or miscommunication) between devices or the misinterpretation of an arbitrary passive reflection.

The handshaking in the identification of the mode of communication can be communicated through a wire between the beacon attached to the secondary instrument and the system driving the imaging catheter. The handshaking can also be communicated ultrasonically by means of a characteristic response from the beacon that is detected in receive mode by the transducer in the imaging catheter. For example, the ultrasonic communication may exist between a primary catheter such as ICE imaging catheter with forward 3D imaging capabilities and an instrument with an optical or higher frequency ultrasonic imaging capability (that can generate a localized ROI of higher resolution), or an instrument of therapeutic persuasion.

Multiple communication frequencies are possible since the ultrasonic communication link can be the result of two 1-way transmission waveforms as opposed to a single two-way transmission based on the transducer in primary intracorporeal ultrasonic imaging probe 100.

For example, two different communication frequencies may be used to simultaneously communicate with two secondary instruments along two different ultrasonic propagation vectors, or one communication operational frequency may be used in Transmit mode of the primary catheter and each beacon of the secondary instrument may transmit with its own ultrasonic waveform signature.
In order for ultrasonic communication to take place, an ultrasonic beacon needs to be positioned within the Field of View of the primary imaging transducer stack. The beacon may be attached to another device as a standalone accessory that requires external wireless power. This wireless power transfer may be by electromagnetic means in the form of inductive coupling or by means of ultrasonic coupling at yet another operational ultrasonic frequency. This ultrasonic harvesting frequency would preferably operate at frequencies of < 10 MHz as most likely the ultrasonic device providing the ultrasonic energy would be on the outside of the body and would need to penetrate fairly deep into the body to communicate with the standalone accessory. The outgoing communication link between the imaging transducer and the beacon is a directed ultrasonic beam.

In some embodiments, non-directional ultrasonic transponders can be included in primary intracorporeal ultrasonic imaging probe 100 for means of establishing a global reference frame, or for other applications. However, it is noted that such non-directional ultrasonic transponders are not required to identify the presence of, and to locate the position of secondary instruments. The incoming communication link may be ultrasonic or may be electric or both.

The ultrasonic communication signal shall be a directional ultrasonic beam that shall be oriented in a direction relative to the imaging direction. This direction may be substantially the same as the imaging direction and in this case, preferably in line with the imaging direction, or it may be along a determined direction that differs from the imaging direction.

A bidirectional ultrasonic communication link may be realized with a beacon that is linked to an external controller over a wire or a fibre optic cable. In this configuration, the beacon receives an ultrasonic signal, the received signal is amplified and brought external to the patient where a controller senses the received signal as a trigger to transmit a characteristic echo signal in return. This characteristic echo signal shall be directed back towards the original imaging signal. The latency in the response of the beacon receiving the signal and then transmitting the return echo can be kept minimal as most of this signal processing takes place in the electrical domain at time scales much faster than ultrasonic propagation.

Multiple beacons can be differentiated by different characteristic signals or waveforms. This differentiation can be used to identify the spatial location of the beacon that has been triggered. Each beacon may have its own wire or fibre or may share wires or fibre.

A group of more than one beacon transducer may be linked to the secondary control and processing system with the use of switches, impedance matching and electrical resonance tuning networks, and ASICs all for the multiplexing and amplifying signals. Although these components may be located within the secondary devices themselves, it will be understood that they may be included in the secondary processing and control system.

Beacon transducers can be made from piezoelectric materials such as piezoelectric ceramics, piezoelectric ceramic and epoxy composite structures, piezoelectric single crystals, relaxor single crystal, relaxor ceramics, relaxor composite structures in a polymer matrix, or polymer piezoelectric layers such as PVDF or PVDF copolymers like P(VDF- TRFE), and can also be made with CMUT and PMUT devices as well.

In some embodiments, a beacon ultrasonic transducer may be powered without requiring a connection to an external controller. This may be achieved, for example, with stored potential electric energy in the form of a battery or may be achieved with actively powering the beacon circuitry by electromagnetic or ultrasonic means. A transcutaneous powering of the beacon may be possible by inductive power or ultrasonic power, and the like. Ultrasonic power may be provided by an auxiliary transducer that is already connected to the control and processing system that controls the primary catheter.

Given that the initial 1-way ultrasonic communication beam is directional and the spatial and temporal characteristics of the ultrasonic communication energy is known relative to the imaging energy, the localization of the secondary instrument in an intracorporeal system of devices can occur without the need for omnidirectional transducers for triangulation (although the use of omnidirectional devices may still be used as beacons on the secondary instrument).

The example embodiments described in the present disclosure can be extended to include one or more beacon circuits attached to primary intracorporeal ultrasonic imaging probe 100 that are in ultrasonic communication with an external tertiary ultrasound imaging probe.

Such an extension represents an example of the geodesic sensor or system required to correct for the variation in the relative directions of the transmitted ultrasonic waveforms if the beacon units are not mounted on a rigid assembly to maintain a fixed orientation between the ultrasonic devices.

The use of separate ultrasonic communication devices from the ultrasonic imaging transducer stack within the primary intracorporeal device is useful if the ultrasonic communication device is able to generate more than one ultrasonic communication A-scan. Such a configuration would help to define the orientation of primary intracorporeal ultrasonic imaging probe 100 relative to other devices (like the said tertiary device) that have established a communication link with primary intracorporeal ultrasonic imaging probe 100. For example, three polymer transducers, each one more distally positioned than the previous one that are embedded within the enclosure of the device such that the transmitter assembly remains stationary relative to the enclosure. This will aid in determining the orientation of primary intracorporeal ultrasonic imaging probe 100 relative to a secondary instrument with multiple beacons by means of triangulation. Another example is to have a single diffraction grating transducer embedded into the enclosure.

## Claims

1. A method of locating a secondary intracorporeal device (110) after performing imaging with an intravascular or intracardiac imaging catheter (100), wherein the imaging catheter comprises an ultrasonic imaging device, and wherein the imaging catheter (100) is configured for three-dimensional scanning, and wherein the secondary intracorporeal device (110) comprises one or more ultrasonic beacon transducers (120) having a combined broad angular response, the method comprising, after performing a scanning operation with the imaging catheter (100) to scan a three-dimensional imaging volume and detect signals associated with received ultrasound imaging energy and communication signals emitted by one or more of the ultrasound beacon transducers (120):
processing signals associated with the received ultrasonic imaging energy to generate an image;
processing the signals to identify a communication signal that was received, when a given imaging A-scan vector was directed towards a given ultrasound beacon transducer (120) on the secondary intracorporeal device (110); and
processing the communication signal to locate the secondary intracorporeal device (110) relative to the imaging catheter (100), based on the direction of the given imaging A-scan vector and a time delay associated with the communication signal.

2. The method according to claim 1 further comprising displaying, on the image, the position of the secondary intracorporeal device (110).

3. The method according to claim 1 or 2 wherein a frequency bandwidth associated with each ultrasonic beacon transducer (120) lies within an imaging bandwidth associated with the ultrasonic imaging device, such that the communication signal is generated in response to the detection, via the given ultrasonic beacon transducer (120), of the emitted ultrasonic imaging energy.

4. The method according to claim 1 or 2 wherein the ultrasonic imaging device is configured to mechanically scan the three-dimensional imaging volume;
wherein the ultrasonic imaging device is further configured to emit ultrasonic communication energy along a plurality of the imaging A-scan vectors;
wherein the ultrasonic communication energy is emitted within a communication frequency band that is different from an imaging frequency band associated with the emitted ultrasonic imaging energy;
wherein the communication signal is generated in response to the detection, via the given ultrasonic beacon transducer (120), of the emitted ultrasonic communication energy.

5. The method according to claim 4 wherein the central frequency of the communication frequency band is greater than the central frequency of the imaging frequency band, and wherein the ultrasonic imaging device has a grating structure formed therein for emitting, for each imaging A-scan vector, ultrasonic communication energy at angles corresponding to at least the first orders of the grating, thereby emitting the ultrasonic communication energy in at least two additional communication A-scan vectors for each imaging A-scan vector.

6. An intracorporeal ultrasonic imaging system comprising:
an intravascular or intracardiac imaging catheter (100) comprising an ultrasonic imaging device, wherein said imaging catheter (100) is configured for scanning a three-dimensional imaging volume;
a secondary intracorporeal device (110) comprising one or more ultrasonic beacon transducers (120) having a combined broad angular response;
a control and processing system interfaced with said imaging catheter (100), said control and processing system comprising one or more processors and memory coupled to said one or more processors, said memory storing instructions, which, when executed by said one or more processors, causes said one or more processors to perform operations comprising:
controlling said imaging catheter (100) to scan the three-dimensional imaging volume such that said ultrasonic imaging device emits ultrasonic imaging energy and receives ultrasonic imaging energy at a plurality of imaging A-scan vectors spanning the three-dimensional imaging volume;
receiving, when a given imaging A-scan vector is directed towards a given ultrasonic beacon transducer (120) on said secondary intracorporeal device (110), a communication signal associated with said given ultrasonic beacon transducer (120);
processing imaging signals associated with the received ultrasonic imaging energy to generate an image; and
processing the communication signal to locate said secondary intracorporeal device (110) relative to said imaging catheter (100), based on the direction of the given imaging A-scan vector and a time delay associated with the communication signal.

7. The intracorporeal ultrasonic imaging system according to claim 6 wherein the control and processing system is further configured to display the image and display the location of the secondary intracorporeal device (110) on the image.

8. The intracorporeal ultrasonic imaging system according to claim 6 or 7 wherein a frequency bandwidth associated with each ultrasonic beacon transducer (120) lies within an imaging bandwidth associated with said ultrasonic imaging device, such that the communication signal is generated in response to the detection, via said given ultrasonic beacon transducer (120), of the emitted ultrasonic imaging energy.

9. The intracorporeal ultrasonic imaging system according to claim 8 wherein said control and processing system is configured to control said secondary intracorporeal device (110) such that said given ultrasonic beacon transducer (120) is operated in echo-backscatter mode, such that the communication signal is an encoded acoustic communication signal backscattered from said given ultrasonic beacon transducer (120) of said secondary intracorporeal device (110) and detected by said ultrasonic imaging device of said imaging catheter (100);
wherein the encoded acoustic communication signal is differentiable from passive backscattered ultrasound imaging signals detected by said ultrasonic imaging device; and
wherein the encoded acoustic communication signal from said given ultrasonic beacon transducer (120) is differentiable from that of other ultrasonic beacon transducers (120) residing at different locations on said secondary intracorporeal device (110).

10. The intracorporeal ultrasonic imaging system according to claim 8 or 9 wherein said ultrasonic imaging device is configured to mechanically scan the three-dimensional imaging volume.

11. The intracorporeal ultrasonic imaging system according to claim 6 or 7 wherein said ultrasonic imaging device is configured to mechanically scan the three-dimensional imaging volume;
wherein said control and processing system is further configured to control said imaging catheter (100) such that said ultrasonic imaging device emits ultrasonic communication energy along a plurality of the imaging A-scan vectors, and such that the ultrasonic communication energy is emitted within a communication frequency band that is different from an imaging frequency band associated with the emitted ultrasonic imaging energy;
wherein the communication signal is generated in response to the detection, via said given ultrasonic beacon transducer (120), of the emitted ultrasonic communication energy.

12. The intracorporeal ultrasonic imaging system according to claim 11 wherein the central frequency of the communication frequency band is greater than the central frequency of the imaging frequency band, and wherein said ultrasonic imaging device has a grating structure formed therein for emitting, for each imaging A-scan vector, ultrasonic communication energy at angles corresponding to at least the first orders of the grating, thereby emitting the ultrasonic communication energy along at least two additional communication A-scan vectors for each imaging A-scan vector.

13. The intracorporeal ultrasonic imaging system according to claim 11 or 12 wherein said ultrasonic imaging device comprises a single-stack ultrasound imaging transducer comprising:
a backing layer;
an inactive piezoelectric layer contacting said backing layer;
a lower electrode layer contacting said inactive piezoelectric layer;
an active piezoelectric layer contacting said lower electrode;
an upper electrode layer contacting said active piezoelectric layer; and
one or more matching layers contacting said upper electrode layer;
wherein the thicknesses of said active piezoelectric layer and said inactive piezoelectric layer are selected to control the frequency of the imaging frequency band and the communication frequency band.

14. The intracorporeal ultrasonic imaging system according to claim 11 or 12 wherein said ultrasonic imaging device comprises a single-stack ultrasound imaging transducer comprising:
a backing layer;
a first lower electrode layer contacting said backing layer;
a first active piezoelectric layer contacting said first lower electrode layer;
a first upper electrode contacting said first active piezoelectric layer;
a first matching layer contacting said first upper electrode;
a second lower electrode contacting said first matching layer;
a second active piezoelectric layer contacting said second lower electrode;
a second upper electrode contacting said second active piezoelectric layer; and
a second matching layer contacting said second upper electrode;
wherein said first active piezoelectric layer has a thickness suitable for defining the imaging frequency band;
wherein said second active piezoelectric layer has a thickness suitable for defining the communication frequency band;
wherein said first matching layer has a thickness approximately equal to a quarter of an operative wavelength associated with the imaging frequency band; and
wherein said second active piezoelectric layer and said second matching layer together have a thickness approximately equal to a quarter of an operational wavelength associated with the imaging frequency band, such that said first matching layer, said second active piezoelectric layer and said second matching layer act as matching layers for said first active piezoelectric layer.

15. The intracorporeal ultrasonic imaging system according to any one of claims 9 to 12 wherein said ultrasonic imaging device comprises an array of ultrasonic transducers that is configured to scan the three-dimensional imaging volume.

## Patentansprüche

1. Verfahren zum Lokalisieren einer sekundären intrakorporalen Vorrichtung (110) nach einem Durchführen einer Bildgebung mit einem intravaskulären oder intrakardialen Bildgebungskatheter (100), wobei der Bildgebungskatheter eine Ultraschallbildgebungsvorrichtung umfasst und wobei der Bildgebungskatheter (100) dafür eingerichtet ist, dreidimensional abzutasten, und wobei die sekundäre intrakorporale Vorrichtung (110) einen oder mehrere Ultraschall-Beacon-Wandler (120) mit einem kombinierten Ansprechverhalten über einem weitem Winkelbereich umfasst, wobei das Verfahren nach dem Durchführen einer Abtastoperation mit dem Bildgebungskatheter (100), um ein dreidimensionales Bildgebungsvolumen abzutasten und Signale, denen die empfangene Ultraschallbildgebungsenergie zugeordnet ist, und Kommunikationssignale, die von einem oder mehreren der Ultraschall-Beacon-Wandler (120) ausgesendet werden, zu erfassen, umfasst:
Verarbeiten von Signalen, denen die empfangene Ultraschallbildgebungsenergie zugeordnet ist, um ein Bild zu erzeugen;
Verarbeiten der Signale, um ein Kommunikationssignal zu identifizieren, das empfangen wurde, wenn ein jeweiliger bildgebender A-Abtastvektor auf einen jeweiligen Ultraschall-Beacon-Wandler (120) an der sekundären intrakorporalen Vorrichtung (110) gerichtet war; und
Verarbeiten des Kommunikationssignals, um die sekundäre intrakorporale Vorrichtung (110) in Bezug auf den Bildgebungskatheter (100) auf der Grundlage der Richtung des jeweiligen bildgebenden A-Abtastvektors und einer mit dem Kommunikationssignal einhergehenden Zeitverzögerung zu lokalisieren.

2. Verfahren nach Anspruch 1, ferner ein Anzeigen der Position der sekundären intrakorporalen Vorrichtung (110) auf dem Bild umfassend.

3. Verfahren nach Anspruch 1 oder 2, wobei die Frequenzbandbreite, die zu jedem Ultraschall-Beacon-Wandler (120) gehört, innerhalb der Bildgebungsbandbreite liegt, die zu der Ultraschallbildgebungsvorrichtung gehört, sodass das Kommunikationssignal in Reaktion auf die mittels des jeweiligen Ultraschall-Beacon-Wandlers (120) erfolgte Erfassung der ausgesendeten Ultraschallbildgebungsenergie erzeugt wird.

4. Verfahren nach Anspruch 1 oder 2, wobei die Ultraschallbildgebungsvorrichtung dafür eingerichtet ist, das dreidimensionale Bildgebungsvolumen mechanisch abzutasten;
wobei die Ultraschallbildgebungsvorrichtung ferner dafür eingerichtet ist, Ultraschallkommunikationsenergie entlang einer Vielzahl der bildgebenden A-Abtastvektoren auszusenden;
wobei die Ultraschallkommunikationsenergie in einem Kommunikationsfrequenzband ausgesendet wird, das sich von dem Bildgebungsfrequenzband, das zu der ausgesendeten Ultraschallbildgebungsenergie gehört, unterscheidet;
wobei das Kommunikationssignal in Reaktion auf die mittels des jeweiligen Ultraschall-Beacon-Wandlers (120) erfolgte Erfassung der ausgesendeten Ultraschallkommunikationsenergie erzeugt wird.

5. Verfahren nach Anspruch 4, wobei die Mittenfrequenz des Kommunikationsfrequenzbandes höher ist als die Mittenfrequenz des Bildgebungsfrequenzbandes, und wobei die Ultraschallbildgebungsvorrichtung eine darin ausgebildete Gitterstruktur aufweist, um für jeden bildgebenden A-Abtastvektor Ultraschallkommunikationsenergie unter Winkeln auszusenden, die mindestens den ersten Ordnungen des Gitters entsprechen, wodurch für jeden bildgebenden A-Abtastvektor die Ultraschallkommunikationsenergie in mindestens zwei zusätzlichen Kommunikations-A-Abtastvektoren ausgesendet wird.

6. Intrakorporales Ultraschallbildgebungssystem, umfassend:
einen intravaskulären oder intrakardialen Bildgebungskatheter (100), der eine Ultraschallbildgebungsvorrichtung umfasst, wobei der Bildgebungskatheter (100) dafür eingerichtet ist, ein dreidimensionales Bildgebungsvolumen abzutasten;
eine sekundäre intrakorporale Vorrichtung (110), die einen oder mehrere Ultraschall-Beacon-Wandler (120) mit einem kombinierten Ansprechverhalten über einem weitem Winkelbereich umfasst;
ein Steuerungs- und Verarbeitungssystem, das über eine Schnittstelle mit dem Bildgebungskatheter (100) verbunden ist, wobei das Steuerungs- und Verarbeitungssystem einen oder mehrere Prozessoren und mit dem einen oder den mehreren Prozessoren gekoppelten Speicher umfasst, wobei der Speicher Anweisungen speichert, die, wenn sie von dem einen oder den mehreren Prozessoren ausgeführt werden, den einen oder die mehreren Prozessoren veranlassen, Operationen auszuführen, die umfassen:
Steuern des Bildgebungskatheters (100), um das dreidimensionale Bildgebungsvolumen abzutasten, derart, dass die Ultraschallbildgebungsvorrichtung Ultraschallbildgebungsenergie aussendet und Ultraschallbildgebungsenergie empfängt, und zwar bei einer Vielzahl von bildgebenden A-Abtastvektoren, die das dreidimensionale Bildgebungsvolumen aufspannen;
Empfangen eines Kommunikationssignals, das zu dem jeweiligen Ultraschall-Beacon-Wandler (120) gehört, wenn ein jeweiliger bildgebender A-Abtastvektor auf einen jeweiligen Ultraschall-Beacon-Wandler (120) an der sekundären intrakorporalen Vorrichtung (110) gerichtet ist;
Verarbeiten von Bildgebungssignalen, denen die empfangene Ultraschallbildgebungsenergie zugeordnet ist,
um ein Bild zu erzeugen; und
Verarbeiten des Kommunikationssignals, um die sekundäre intrakorporale Vorrichtung (110) in Bezug auf den Bildgebungskatheter (100) auf der Grundlage der Richtung des jeweiligen bildgebenden A-Abtastvektors und einer mit dem Kommunikationssignal einhergehenden Zeitverzögerung zu lokalisieren.

7. Intrakorporales Ultraschallbildgebungssystem nach Anspruch 6, wobei das Steuerungs- und Verarbeitungssystem ferner dafür eingerichtet ist, das Bild anzuzeigen und den Ort der sekundären intrakorporalen Vorrichtung (110) auf dem Bild anzuzeigen.

8. Intrakorporales Ultraschallbildgebungssystem nach Anspruch 6 oder 7, wobei die Frequenzbandbreite, die zu jedem Ultraschall-Beacon-Wandler (120) gehört, innerhalb der Bildgebungsbandbreite liegt, die zu der Ultraschallbildgebungsvorrichtung gehört, sodass das Kommunikationssignal in Reaktion auf die mittels des jeweiligen Ultraschall-Beacon-Wandlers (120) erfolgte Erfassung der ausgesendeten Ultraschallbildgebungsenergie erzeugt wird.

9. Intrakorporales Ultraschallbildgebungssystem nach Anspruch 8, wobei das Steuerungs- und Verarbeitungssystem dafür eingerichtet ist, die sekundäre intrakorporale Vorrichtung (110) so zu steuern, dass der jeweilige Ultraschall-Beacon-Wandler (120) im Echo-Backscatter-Modus betrieben wird, so dass das Kommunikationssignal ein codiertes akustisches Kommunikationssignal ist, das von dem jeweiligen Ultraschall-Beacon-Wandler (120) der sekundären intrakorporalen Vorrichtung (110) rückgestreut und von der Ultraschallbildgebungsvorrichtung des Bildgebungskatheters (100) erfasst wird;
wobei das codierte akustische Kommunikationssignal von passiven rückgestreuten Ultraschallbildgebungssignalen, die von der Ultraschallbildgebungsvorrichtung erfasst werden, unterscheidbar ist und
wobei das codierte akustische Kommunikationssignal von dem jeweiligen Ultraschall-Beacon-Wandler (120) von denen anderer Ultraschall-Beacon-Wandler (120), die sich an verschiedenen Orten an der sekundären intrakorporalen Vorrichtung (110) befinden, unterscheidbar ist.

10. Intrakorporales Ultraschallbildgebungssystem nach Anspruch 8 oder 9, wobei die Ultraschallbildgebungsvorrichtung dafür eingerichtet ist, das dreidimensionale Bildgebungsvolumen mechanisch abzutasten.

11. Intrakorporales Ultraschallbildgebungssystem nach Anspruch 6 oder 7, wobei die Ultraschallbildgebungsvorrichtung dafür eingerichtet ist, das dreidimensionale Bildgebungsvolumen mechanisch abzutasten;
wobei das Steuerungs- und Verarbeitungssystem ferner dafür eingerichtet ist, den Bildgebungskatheter (100) so zu steuern, dass die Ultraschallbildgebungsvorrichtung Ultraschallkommunikationsenergie entlang einer Vielzahl der bildgebenden A-Abtastvektoren aussendet, und so, dass die Ultraschallkommunikationsenergie innerhalb eines Kommunikationsfrequenzbandes ausgesendet wird, das sich von einem Bildgebungsfrequenzband, das zu der ausgesendeten Ultraschallbildgebungsenergie gehört, unterscheidet;
wobei das Kommunikationssignal in Reaktion auf die mittels des jeweiligen Ultraschall-Beacon-Wandlers (120) erfolgte Erfassung der ausgesendeten Ultraschallkommunikationsenergie erzeugt wird.

12. Intrakorporales Ultraschallbildgebungssystem nach Anspruch 11, wobei die Mittenfrequenz des Kommunikationsfrequenzbandes höher ist als die Mittenfrequenz des Bildgebungsfrequenzbandes und wobei die Ultraschallbildgebungsvorrichtung eine Gitterstruktur aufweist, die darin ausgebildet ist, um für jeden bildgebenden A-Abtastvektor Ultraschallkommunikationsenergie unter Winkeln auszusenden, die mindestens den ersten Ordnungen des Gitters entsprechen, wodurch für jeden bildgebenden A-Abtastvektor die Ultraschallkommunikationsenergie entlang von mindestens zwei zusätzlichen Kommunikations-A-Abtastvektoren ausgesendet wird.

13. Intrakorporales Ultraschallbildgebungssystem nach Anspruch 11 oder 12, wobei die Ultraschallbildgebungsvorrichtung einen Einzelstapel-Ultraschallbildgebungswandler umfasst, der Folgendes umfasst:
eine Trägerschicht;
eine inaktive piezoelektrische Schicht, die mit der Trägerschicht in Kontakt steht;
eine untere Elektrodenschicht, die mit der inaktiven piezoelektrischen Schicht in Kontakt steht;
eine aktive piezoelektrische Schicht, die mit der unteren Elektrode in Kontakt steht;
eine obere Elektrodenschicht, die mit der aktiven piezoelektrischen Schicht in Kontakt steht;
und
eine oder mehrere Anpassungsschichten, die mit der oberen Elektrodenschicht in Kontakt stehen;
wobei die Dicken der aktiven piezoelektrischen Schicht und der inaktiven piezoelektrischen Schicht ausgewählt sind, um die Frequenz des Bildgebungsfrequenzbandes und des Kommunikationsfrequenzbandes einzustellen.

14. Intrakorporales Ultraschallbildgebungssystem nach Anspruch 11 oder 12, wobei die Ultraschallbildgebungsvorrichtung einen Einzelstapel-Ultraschallbildgebungswandler umfasst, der Folgendes umfasst:
eine Trägerschicht;
eine erste untere Elektrodenschicht, die mit der Trägerschicht in Kontakt steht;
eine erste aktive piezoelektrische Schicht, die mit der ersten unteren Elektrodenschicht in Kontakt steht;
eine erste obere Elektrode, die mit der ersten aktiven piezoelektrischen Schicht in Kontakt steht;
eine erste Anpassungsschicht, die mit der ersten oberen Elektrode in Kontakt steht;
eine zweite untere Elektrode, die mit der ersten Anpassungsschicht in Kontakt steht;
eine zweite aktive piezoelektrische Schicht, die mit der zweiten unteren Elektrode in Kontakt steht;
eine zweite obere Elektrode, die mit der zweiten aktiven piezoelektrischen Schicht in Kontakt steht; und
eine zweite Anpassungsschicht, die mit der zweiten oberen Elektrode in Kontakt steht;
wobei die erste aktive piezoelektrische Schicht eine Dicke aufweist, die geeignet ist, das Bildgebungsfrequenzband zu definieren;
wobei die zweite aktive piezoelektrische Schicht eine Dicke aufweist, die geeignet ist, das Kommunikationsfrequenzband zu definieren;
wobei die erste Anpassungsschicht eine Dicke aufweist, die ungefähr gleich einem Viertel einer Betriebswellenlänge ist, die dem Bildgebungsfrequenzband zugeordnet ist; und
wobei die zweite aktive piezoelektrische Schicht und die zweite Anpassungsschicht zusammen eine Dicke aufweisen, die ungefähr einem Viertel einer Betriebswellenlänge ist, die dem Bildgebungsfrequenzband zugeordnet ist, sodass die erste Anpassungsschicht, die zweite aktive piezoelektrische Schicht und die zweite Anpassungsschicht als Anpassungsschichten für die erste aktive piezoelektrische Schicht wirksam werden.

15. Intrakorporales Ultraschallbildgebungssystem nach einem der Ansprüche 9 bis 12, wobei die Ultraschallbildgebungsvorrichtung eine Anordnung von Ultraschallwandlern umfasst, die dafür eingerichtet ist, das dreidimensionale Bildgebungsvolumen abzutasten.

## Revendications

1. Procédé de localisation d'un dispositif intracorporel secondaire (110) après avoir effectué une imagerie avec un cathéter d'imagerie intravasculaire ou intracardiaque (100), dans lequel le cathéter d'imagerie comprend un dispositif d'imagerie ultrasonore et dans lequel le cathéter d'imagerie (100) est configuré pour un balayage tridimensionnel et dans lequel le dispositif intracorporel secondaire (110) comprend au moins un transducteur de balise ultrasonore (120) à large réponse angulaire combinée, le procédé comprenant, après avoir effectué une opération de balayage avec le cathéter d'imagerie (100), le balayage d'un volume d'imagerie tridimensionnelle et la détection des signaux associés à l'énergie d'imagerie ultrasonore reçue et aux signaux de communication émis par au moins un transducteur de balise ultrasonore (120) :
le traitement des signaux associés à l'énergie d'imagerie ultrasonore reçue pour générer une image ;
le traitement des signaux pour identifier un signal de communication reçu, lorsqu'un vecteur donné de balayage A d'imagerie a été dirigé vers un transducteur donné de balise ultrasonore (120) sur le dispositif intracorporel secondaire (110) ; et
le traitement du signal de communication pour localiser le dispositif intracorporel secondaire (110) par rapport au cathéter d'imagerie (100), sur la base de la direction du vecteur donné de balayage A d'imagerie et d'un retard associé au signal de communication.

2. Procédé selon la revendication 1, comprenant en outre l'affichage, sur l'image, de la position du dispositif intracorporel secondaire (110).

3. Procédé selon la revendication 1 ou 2, dans lequel une largeur de bande de fréquences associée à chaque transducteur de balise ultrasonore (120) se situe dans une largeur de bande d'imagerie associée au dispositif d'imagerie ultrasonore, de sorte que le signal de communication soit généré en réponse à la détection, par le transducteur donné de balise ultrasonore (120), de l'énergie d'imagerie ultrasonore émise.

4. Procédé selon la revendication 1 ou 2, dans lequel le dispositif d'imagerie ultrasonore est conçu pour balayer mécaniquement le volume d'imagerie tridimensionnelle ;
dans lequel le dispositif d'imagerie ultrasonore est en outre conçu pour émettre de l'énergie de communication ultrasonore le long d'une pluralité des vecteurs de balayage A d'imagerie ;
dans lequel l'énergie de communication ultrasonore est émise dans une bande de fréquences de communication qui diffère d'une bande de fréquences d'imagerie associée à l'énergie d'imagerie ultrasonore émise ;
dans lequel le signal de communication est généré en réponse à la détection, par le transducteur donné de balise ultrasonore (120), de l'énergie émise de communication ultrasonore.

5. Procédé selon la revendication 4, dans lequel la fréquence centrale de la bande de fréquences de communication est supérieure à la fréquence centrale de la bande de fréquences d'imagerie et dans lequel le dispositif d'imagerie ultrasonore a une structure de réseau qui y est formée pour émettre, pour chaque vecteur de balayage A d'imagerie, de l'énergie de communication ultrasonore à des angles correspondant au moins aux premiers ordres du réseau, en émettant ainsi l'énergie de communication ultrasonore dans au moins deux vecteurs supplémentaires de balayage A de communication pour chaque vecteur de balayage A d'imagerie.

6. Système d'imagerie ultrasonore intracorporelle comprenant :
un cathéter d'imagerie intravasculaire ou intracardiaque (100) comprenant un dispositif d'imagerie ultrasonore, ledit cathéter d'imagerie (100) étant conçu pour balayer un volume d'imagerie tridimensionnelle ;
un dispositif intracorporel secondaire (110) comprenant au moins un transducteur de balise ultrasonore (120) présentant une large réponse angulaire combinée ;
un système de commande et de traitement interfacé avec ledit cathéter d'imagerie (100), ledit système de commande et de traitement comprenant au moins un processeur et une mémoire couplée audit au moins un processeur, ladite mémoire stockant des instructions qui, lorsqu'elles sont exécutées par ledit au moins un processeur, amènent ledit au moins un processeur à effectuer des opérations comprenant :
la commande dudit cathéter d'imagerie (100) pour balayer le volume d'imagerie tridimensionnel de sorte que ledit dispositif d'imagerie ultrasonore émette de l'énergie d'imagerie ultrasonore et reçoive de l'énergie d'imagerie ultrasonore au niveau d'une pluralité de vecteurs de balayage A d'imagerie couvrant le volume d'imagerie tridimensionnelle ;
la réception, lorsqu'un vecteur de balayage A d'imagerie donné est dirigé vers un transducteur donné de balise ultrasonore (120) sur ledit dispositif intracorporel secondaire (110), d'un signal de communication associé audit transducteur donné de balise ultrasonore (120) ;
le traitement des signaux d'imagerie associés à l'énergie d'imagerie ultrasonore reçue
afin de générer une image ; et
le traitement du signal de communication pour localiser ledit dispositif intracorporel secondaire (110) par rapport audit cathéter d'imagerie (100), en fonction de la direction du vecteur donné de balayage A d'imagerie et d'un retard associé au signal de communication.

7. Système d'imagerie ultrasonore intracorporelle selon la revendication 6, dans lequel le système de commande et de traitement est en outre configuré pour afficher l'image et pour afficher l'emplacement du dispositif intracorporel secondaire (110) sur l'image.

8. Système d'imagerie ultrasonore intracorporelle selon la revendication 6 ou 7, dans lequel une largeur de bande de fréquences associée à chaque transducteur de balise ultrasonore (120) se trouve dans une largeur de bande d'imagerie associée audit dispositif d'imagerie ultrasonore, de sorte que le signal de communication soit généré en réponse à la détection, par ledit transducteur de balise ultrasonore donné (120), de l'énergie émise d'imagerie ultrasonore.

9. Système d'imagerie ultrasonore intracorporelle selon la revendication 8, dans lequel ledit système de commande et de traitement est configuré pour commander ledit dispositif intracorporel secondaire (110) de sorte que ledit transducteur donné de balise ultrasonore (120) fonctionne en mode d'écho-rétrodiffusion, de sorte que le signal de communication soit un signal de communication acoustique codé rétrodiffusé dudit transducteur donné de balise ultrasonore (120) dudit dispositif intracorporel secondaire (110) et détecté par ledit dispositif d'imagerie ultrasonore dudit cathéter d'imagerie (100) ;
dans lequel le signal de communication acoustique codé est différentiable des signaux d'imagerie ultrasonore rétrodiffusés passifs détectés par ledit dispositif d'imagerie ultrasonore ; et
dans lequel le signal de communication acoustique codé provenant dudit transducteur donné de balise ultrasonore (120) est différentiable de celui d'autres transducteurs de balise ultrasonore (120) se trouvant à différents endroits sur ledit dispositif intracorporel secondaire (110).

10. Système d'imagerie ultrasonore intracorporelle selon la revendication 8 ou 9, dans lequel ledit dispositif d'imagerie ultrasonore est conçu pour balayer mécaniquement le volume d'imagerie tridimensionnelle.

11. Système d'imagerie ultrasonore intracorporelle selon la revendication 6 ou 7, dans lequel ledit dispositif d'imagerie ultrasonore est conçu pour balayer mécaniquement le volume d'imagerie tridimensionnelle ;
dans lequel ledit système de commande et de traitement est en outre configuré pour commander ledit cathéter d'imagerie (100) de sorte que ledit dispositif d'imagerie ultrasonore émette de l'énergie de communication ultrasonore le long d'une pluralité des vecteurs de balayage A d'imagerie et de sorte que l'énergie de communication ultrasonore soit émise dans une bande de fréquences de communication différente d'une bande de fréquences d'imagerie associée à l'énergie d'imagerie ultrasonore émise ;
dans lequel le signal de communication est généré en réponse à la détection, par ledit transducteur de balise ultrasonore donné (120), de l'énergie de communication ultrasonore émise.

12. Système d'imagerie ultrasonore intracorporelle selon la revendication 11, dans lequel la fréquence centrale de la bande de fréquences de communication est supérieure à la fréquence centrale de la bande de fréquences d'imagerie et dans lequel ledit dispositif d'imagerie ultrasonore a une structure de réseau qui y est formée pour émettre, pour chaque vecteur de balayage A d'imagerie, de l'énergie de communication ultrasonore à des angles correspondant au moins aux premiers ordres du réseau, en émettant ainsi l'énergie de communication ultrasonore le long d'au moins deux vecteurs supplémentaires de balayage A de communication pour chaque vecteur de balayage A d'imagerie.

13. Système d'imagerie ultrasonore intracorporelle selon la revendication 11 ou 12, dans lequel ledit dispositif d'imagerie ultrasonore comprend un transducteur d'imagerie ultrasonore à empilement unique comprenant :
une couche de support ;
une couche piézoélectrique inactive, en contact avec ladite couche de support ;
une couche d'électrode inférieure, en contact avec ladite couche piézoélectrique inactive ;
une couche piézoélectrique active, en contact avec ladite électrode inférieure ;
une couche d'électrode supérieure, en contact avec ladite couche piézoélectrique active ;
et
au moins une couche d'adaptation, en contact avec ladite couche d'électrode supérieure ;
dans lequel les épaisseurs de ladite couche piézoélectrique active et de ladite couche piézoélectrique inactive sont sélectionnées pour commander la fréquence de la bande de fréquences d'imagerie et de la bande de fréquences de communication.

14. Système d'imagerie ultrasonore intracorporelle selon la revendication 11 ou 12, dans lequel ledit dispositif d'imagerie ultrasonore comprend un transducteur d'imagerie ultrasonore à empilement unique comprenant :
une couche de support ;
une première couche d'électrode inférieure, en contact avec ladite couche de support ;
une première couche piézoélectrique active, en contact avec ladite première couche d'électrode inférieure ;
une première électrode supérieure, en contact avec ladite première couche piézoélectrique active ;
une première couche d'adaptation, en contact avec ladite première électrode supérieure ;
une seconde électrode inférieure, en contact avec ladite première couche d'adaptation ;
une seconde couche piézoélectrique active, en contact avec ladite seconde électrode inférieure ;
une seconde électrode supérieure, en contact avec ladite seconde couche piézoélectrique active ; et
une seconde couche d'adaptation, en contact avec ladite seconde électrode supérieure ;
dans lequel ladite première couche piézoélectrique active a une épaisseur appropriée pour définir la bande de fréquences d'imagerie ;
dans lequel ladite seconde couche piézoélectrique active a une épaisseur appropriée pour définir la bande de fréquences de communication ;
dans lequel ladite première couche d'adaptation a une épaisseur approximativement égale au quart d'une longueur d'onde opérationnelle associée à la bande de fréquences d'imagerie ; et
dans lequel ladite seconde couche piézoélectrique active et ladite seconde couche d'adaptation ont ensemble une épaisseur approximativement égale au quart d'une longueur d'onde opérationnelle associée à la bande de fréquences d'imagerie, de sorte que ladite première couche d'adaptation, ladite seconde couche piézoélectrique active et ladite seconde couche d'adaptation agissent comme couches d'adaptation pour ladite première couche piézoélectrique active.

15. Système d'imagerie ultrasonore intracorporelle selon l'une quelconque des revendications 9 à 12, dans lequel ledit dispositif d'imagerie ultrasonore comprend un réseau de transducteurs ultrasonores qui est conçu pour balayer le volume d'imagerie tridimensionnelle.
